# EUROPEAN PATENT SPECIFICATION

(11) **EP 1 598 017 B1**
(45) Date of publication and mention of the grant of the patent: **12.04.2017**
(21) Application number: 04714882.0
(22) Date of filing: 26.02.2004
(51) Int. Cl.: A61B 17/04

(54) **MEDICAL INSTRUMENT**
MEDIZINISCHES INSTRUMENT
INSTRUMENT MEDICAL

(30) Priority: 26.02.2003 JP 2003048883; 09.02.2004 JP 2004032721
(43) Date of publication of application: 23.11.2005
(73) Proprietor: SUMITOMO BAKELITE CO., LTD., Tokyo 140-0002 (JP); Suzuki, Yutaka, Minato-ku, Tokyo 105-0002 (JP)
(72) Inventor: Suzuki, Yutaka, Tokyo 105-0002 (JP); Kojo, Yasunori, c/o Akita Sumitomo Bake Co., Ltd., Akita-shi, Akita 011-8510 (JP); Arikawa, Kiyotaka, c/o Akita Sumitomo Bake Co. Ltd, Akita-shi, Akita 011-8510 (JP); Sakuguchi, Yukihiko, Akita Sumitomo Bake Co., Ltd., Akita-shi, Akita 011-8510 (JP)
(74) Representative: Schollweck, Susanne
(86) International application number: PCT/JP2004/002253
(87) International publication number: WO 2004/075761

(56) References cited:
- WO-A1-02/39905
- JP-A- 5 161 655
- JP-A- 5 161 655
- JP-A- 2003 225 240
- JP-B2- 6 024 533
- US-A- 5 330 488
- US-A- 5 722 981
- US-A1- 2002 156 344
- US-A1- 2003 004 544

## Description

### Field of the invention

The present invention relates to a medical device.

### Description of the related art

To feed a patient who cannot take nutrients from his or her mouth, three feeding methods have been adopted: intravenous feeding, gastric feeding using a tube inserted from a nose into a stomach, and enteral feeding from grastric fistula.

In recent years, by the growth of enteral nutritional products and application methods therefor, enteral nutrition management by means of percutaneous endoscopic gastrostomy (PEG) has been frequently conducted. According to the PEG, a gastrostomy is made with a less complicated operation than that by surgical gastronomy, so the PEG causes small invasion and costs far less than the surgical gastronomy. Thus, the PEG is considered standard of the gastrostomy in the Western countries.

According to the PEG, a through hole that penetrates an abdominal wall and a stomach wall of a patient is formed prior to the gastrostomy. Prior to formation of the through hole, the stomach wall which tends to move is temporarily fixed. Normally, therefore, the abdominal wall and the stomach wall are put in a suture.

As medical devices for such suturing operation, a medical device consisting of, for example, a suture insertion needle, a suture grasping needle arranged in parallel to the suture insertion needle, a stylet slidably inserted into the suture grasping needle, and a fixing member that fixes the suture insertion needle and the suture grasping needle on their respective base ends, and configured so that the suture is grasped by the stylet is disclosed (Patent Literature 1).

The medical device disclosed in the Patent Literature 1 includes (a) the hollow suture insertion needle having an internal passage into which a suture is inserted, (b) the hollow suture grasping needle arranged in parallel therto and used to grasp the suture, (c) the stylet slidably inserted into the suture grasping needle, and (d) the fixing member fixing the suture insertion needle and the suture grasping needle on their respective base ends. This stylet includes an annular member which consists of an elastic material, which can be stored in the internal passage of the suture grasping needle, and which is provided on a tip end of the stylet. The annular member is configured to extend toward the suture insertion needle while protruding from the tip end of the suture grasping needle so that a central axis of the suture insertion needle or an extension thereof is passed through an inside of the annular member.

According to the Patent Literature 1, this medical device is used as follows. First, a living body is punctured by the suture insertion needle and the suture grasping needle. The stylet is inserted into the suture grasping needle from a rear end thereof, and the annular member protrudes from the tip end of the suture grasping needle. The suture is inserted into the suture insertion needle from a rear end thereof. As a result, the suture is exposed from the tip end of the suture insertion needle. In this' state, the stylet within the suture grasping needle is moved backward, whereby the suture is grasped by the annular member. Finally, the suture insertion needle, the suture grasping needle, and the stylet are integrally moved backward from the living body. One end of the suture is thereby pulled out from the suture insertion needle side and the other end thereof is pulled out from the suture grasping needle.

However, when the medical device disclosed in the Patent Literature 1 is used, the suture is captured by the annular member on the tip end of the suture grasping needle, which tip end is sharp. If the suture contacts with the suture grasping needle by excessively strong force when the stylet is moved backward, the suture may possibly be cut off.

Further, with the medical device disclosed in the Patent Literature 1, the two puncture needles should be used simultaneously to puncture the living body. Due to this, more advanced expertise is required as compared with operation of puncturing the living body with one needle.

To ensure fixing the abdominal wall to the stomach wall using the medical device, it is necessary to perform the same suturing operation twice, which is disadvantageously a burden on the patient.

To prevent this disadvantage, a suture instrument for a living body is disclosed in Patent Literature 2. This living body suture instrument includes (a) a hollow fixing needle including an internal passage into which a suture is inserted and having a sharp tip end, (b) a hollow outer cylinder arranged in parallel to the hollow fixing needle, including an internal passage into which the suture is inserted, and having a non-sharp tip end, (c) a movable needle which can be selectively pulled out from the internal passage of the outer cylinder and having a sharp tip end, and a suture traction tool, and (d) a holder holding the fixing needle and the outer cylinder in parallel at one of their positions, respectively.

According to the Patent Literature 2, this living body suture instrument solves the above-stated disadvantage. First, a living body is punctured by the fixing needle and the movable needle inserted into the outer cylinder. The movable needle is then pulled out from the outer cylinder, the suture traction tool is inserted into the outer cylinder from a rear end thereof, and a snare protrudes from a tip end of the outer cylinder. The suture is inserted into the fixing needle from a rear end thereof. As a result, the suture is exposed from a tip end of the fixing needle. In this state, the suture traction tool within the outer cylinder is moved backward, whereby the suture is pulled out from the outer cylinder to the outside of the living body through the non-sharp tip end while the suture is grasped by the snare. At this time, it is not necessary for the suture to pass through the sharp portion, so that the suture is prevented from being cut off.

However, according to the living body suture instrument disclosed in the Patent Literature 2, since the suture traction tool is passed through an inside of the outer cylinder while grasping the suture, a diameter of the outer cylinder becomes disadvantageously large. Besides, even if the movable needle is sharp, the puncture performance may possibly be deteriorated by influence of a difference in height between the movable needle and the outer cylinder.

Moreover, according to the living body suture instrument disclosed in the Patent Literature 2, it is required to simultaneously operate the two needles although they are not used to simultaneously puncture the living body. Due to this, more advanced expertise is required as compared with operation of puncturing the living body with one needle.

Also, the suture insertion needle and the suture grasping needle of the medical device in the patent literature 1 and the fixing needle of the suture instrument for the living body in the patent literature 2 are hollow needle, so the puncture performances of the needles have tend to deteriorate relative to the case with a non-hollow needle.
Patent Literature 1: Japanese Patent Application Laid-Open No. H4-226643
Patent Literature 2: Japanese Patent Application Laid-Open No. 2003-225240.

Document JP 5-161655A discloses a suturing device which is considered to be the closest prior art.

### SUMMARY OF THE INVENTION

The present invention has been achieved in view of these circumstances. It is an object of the present invention to provide a medical device, according to independent claims 1 or 9, capable of improving operativity and safety when suturing a body surface part and an internal tissue.

According to the present invention, there is provided a medical device comprising:
(a) a suture insertion needle that is a hollow puncture needle including a sharp tip end, the tip end being non-hollow, that includes a lateral hole near the tip end, and that is used by inserting a suture into a hollow portion of the suture insertion needle;
(b) a suture grasping needle that is a hollow puncture needle arranged sideways of the suture insertion needle substantially in parallel to the suture insertion needle and including a sharp tip end, the tip end being non-hollow, that includes a lateral hole near the tip end, and that is used by inserting the suture into a hollow portion of the suture grasping needle;
(c) a suture traction tool slidably inserted into the hollow portion of the suture grasping needle;
(d) a fixing member for relatively determining a tip end position of the suture insertion needle and a tip end position of the suture grasping needle; and
(e) an abutment member that supports the suture insertion needle and the suture grasping needle, and that abuts on a suture target; and
(f) a notch being formed form a side edge deep into said abutment member.

In addition, according to the present invention, there is provided a medical device used to put a body surface part and an internal tissue in a suture, comprising: a main body; a suture insertion needle which is supported by the main body, and into which the suture can be inserted; and a suture grasping needle which is provided to be proximate to the suture insertion needle, and into which a suture traction tool can be inserted, wherein the suture grasping needle includes a sharp tip end and an opening provided in proximity to a base end side relative to the tip end, and is configured so that the suture traction tool can be withdrawn from the opening to an outside of the suture grasping needle.

In the medical device according to the present invention, the tip ends of the respective puncture needles are non-hollow, so that the puncture performances of the needles can be improved. The hollow portion of each puncture needle communicates with the outside thereof through the lateral hole or the opening near the tip end. Due to this, it is possible to ensure that the suture and the suture traction tool are pulled out from the lateral hole to a predetermined direction. Thus, good operativity and safe suturing operation can be ensured. In addition, since the suture insertion needle and the suture grasping needle are supported by the abutment member and the abutment member abuts on suture targets such as the body surface part, the suturing operation can be ensured.

In the medical device according to the present invention, the suture traction tool may include a snare provided on a tip end of said suture traction tool and formed out of an elastically deformable material, which extends so that an extension of the suture insertion needle passes through an inside of the snare in a free state, and can be stored in the hollow portion of the suture grasping needle in an elastically deformed state. By configuring so, the suture can be surely grasped. The suturing operation can be therefore ensured.

In the medical device according to the present invention, the abutment member may support the suture insertion needle and the suture grasping needle substantially parallel to each other. By configuring so, the suturing operation can be stably performed.

In the medical device according to the present invention, the fixing member may be provided at the suture grasping needle, and configured to be able to determine the tip end position of the suture insertion needle relative to the tip end position of the suture grasping needle, to an arbitrary position. By configuring so, the operativity during the suturing operation can be further improved.

In the medical device according to the present invention, a notch configured to remove the suture from the abutment member and the fixing member may be provided at each of the abutment member-and the fixing member. By configuring so, while the suture grasping needle punctures the body, it is possible to ensure that the suture is removed from the abutment member and the fixing member. It is, therefore, possible to ensure performing the suturing by simple operation.

In the medical device according to the present invention, an edge of the lateral hole of the suture insertion needle and an edge of the lateral hole of the suture grasping needle may be chamfered to have a C-shaped surface or an R-shaped surface. This make it possible to suppress breaking of the suture. It is, therefore, possible to further ensure the suturing operation.

In themedicaldevice according to the present invention, at least one of the suture insertion needle and the suture grasping needle can be detachably supported by the abutment member. By configuring so, the suturing operation can be further simplified

The medical device according to the present invention may further comprise a plurality of the suture insertion needles, and the suture grasping needle may be configured to be provided to be proximate to each of the plurality of suture insertion needles. Thus, the operation of removing the suture insertion needle and the operation of rotating the abutment member when suturing a plurality of sites are possible to be not required. It is, therefore, possible to further ensure that the plurality of sites are stably sutured by the simple operation.

According to the present invention, there is provided a medical device used for suturing a body surface part and an internal tissue in a suture, comprising: a main body; a suture insertion needle which is supported by the main body, and into which the suture can be inserted; and a suture grasping needle which is provided to be proximate to the suture insertion needle, and into which a suture traction tool can be inserted, whrein at least one of the suture insertion needle and the suture grasping needle is detachably attached to the main body.

In the medical device according to the present invention, at least one of the suture insertion needle and the suture grasping needle is detachably attached to the main body. It is, therefore, possible to ensure good operativity and stable suturing operation.

In the medical device according to the present invention, the main body may include a fixing member that fixes one of a tip end of the suture insertion needle and a tip end of the suture grasping needle to a predetermined position; and a support member that supports the fixing member. By configuring so, stable suturing operation can be ensured.

In the medical device according to the present invention, the main body may further include an abutment member that is provided on a tip end side of the support member, that abuts on the body surface part, and that supports the suture insertion needle and the suture grasping needle. By configuring so, the medical device can abut on the body surface part at the abutment member thereof during the suturing operation. Thus, it is possible to further ensure the stable suturing operation. In the medical device according to the present invention, the support member may be configured to support the fixing member and the abutment member. By configuring so, the suture insertion needle and the suture gasping needle can be supported by the support member through the abutment member. It is, therefore, possible to further ensure supporting these puncture needles and further improve the stability of the medical device during use.

In the medical device according to the present invention, the suture grasping needle may be configured so that a guide wire can be also inserted into the suture grasping needle. By configuring so, an applicable range of the medical device can be enlarged. For example, if a guide wire is used, it is possible to appropriately determine a catheter insertion route and to insert a catheter safely and surely. Further, if the guide wire is used, it is possible to realize insertion of the guide wire simultaneously with suturing the body surface part and the internal tissue. Due to this, it is possible to appropriately determine the catheter insertion route after the suturing operation and to insert the catheter safely and surely. Besides, the number of times by which the puncture needles puncture the body surface and the internal tissue until the catheter is inserted can be reduced, the invasion of the needles into the patient occurs quite infrequently, and the patient is less burdened. It is, therefore, possible to return the patient to ordinary life at earlier time and to reduce medical cost of the patient.

In the medical device according to the present invention, an outside diameter of the guide wire may be equal to or larger than 0.1 mm and equal to or smaller than 0.8 mm.

In the medical device according to the present invention, the fixing member may include a notch that makes one of the suture insertion needle and the suture grasping needle detachable from the main body. By configuring so, it is possible to ensure attachment and detachment of the suture insertion needle or the suture grasping needle.

In the medical device according to the present invention, the abutment member includes a notch that makes one of the suture insertion needle and the suture grasping needle detachable from the main body. By configuring so, the attachment and detachment of the suture insertion needle and the suture grasping needle can be performed simply and surely.

In the medical device according to the present invention, the suture insertion needle and the suture grasping needle may be configured to be able to puncture independently of each other. By configuring so, the two puncture needles can be operated individually without puncturing simultaneously by the two puncture needles or without simultaneously operating the two puncture needles. Therefore, good operativity and safe operation can be ensured. In addition, the stress of the patient can be lessened and operation time can be shortened. Itis, therefore, possible to improve the operativity and safety when suturing the body surface part and the internal tissue by the suture.

In the medical device according to the present invention, while the suture insertion needle and the suture grasping needle puncture and the main body abuts on the body surface part, the suture insertion needle can be independently removed. By configuring so, the suture insertion needle can be removed while grasping the suture by the suture grasping needle. It is, therefore, possible to suture a plurality of sites by a series of operations.

In the medical device according to the present invention, the main body may be rotatable about the suture grasping needle used as a rotational axis. By configuring so, it is possible to ensure suturing a plurality of sites.

The medical device according to the present invention includes a plurality of the suture insertion needles, and the suture grasping needle can be provided to be proximate to each of the plurality of suture insertion needles. By configuring so, the operation of removing the suture insertion needle alone and the operation of rotating the main body when suturing a plurality of sites are possible to be not required. It is, therefore, possible to further ensure that the plurality of sites is sutured by the simple operation.

Arbitrary combinations of these respective constituent elements are also effective as the modes of the present invention.

### BRIEF DESCRIPTION OF THE DRAWINGS

The above object, the other objects, features and advantages of the invention will be readily apparent from the following preferred embodiments and accompanying drawings.
Fig. 1 is a sectional front view that depicts a medical device according to one embodiment of the present invention.
Fig. 2a is a sectional front view that depicts a configuration of a suture grasping needle of the medical device according to the embodiment.
Fig. 2b is a sectional view that depicts a configuration of a suture traction tool of the medical device according to the embodiment.
Fig. 2c is a sectional front view that depicts a configuration of a combination of Figs. 2a and 2b.
Fig. 3 is a sectional front view of a suture insertion needle of the medical device according to the embodiment.
Fig. 4 is a sectional front vi.ew that depicts a tip end of the suture grasping needle of the medical device according to the embodiment.
Fig. 5 depicts a front view and a plan view of an abutment member of the medical device according to the embodiment.
Fig. 6 is an explanatory view for exemplary procedures for using the medical device according to the embodiment.
Fig. 7 is a sectional front view that depicts a step 1 of an exemplary method for using the medical procedure shown in Fig. 6.
Fig. 8 is a sectional front view that depicts a step 2 of the exemplary method for using the medical procedure shown in Fig. 6.
Fig. 9 is a sectional front view that depicts a step 3 of the exemplary method for using the medical procedure shown in Fig. 6.
Fig 10 is a sectional front view that depicts a step 4 of the exemplary method for using the medical procedure shown in Fig. 6.
Fig. 11 is a sectional front view that depicts a step 5 of the exemplary method for using the medical procedure shown in Fig. 6.
Fig. 12 is a sectional front view that depicts a step 6 of the exemplary method for using the medical procedure shown in Fig. 6.
Fig. 13 is a sectional front view that depicts a step 7 of the exemplary method for using the medical procedure shown in Fig. 6.
Fig. 14 is a sectional front view that depicts a step 8 of the exemplary method for using the medical procedure shown in Fig. 6.
Fig. 15 is a sectional front view that depicts a step 9 of the examplary method for using the medical procedure shown in Fig. 6.
Fig. 16 is a sectional front view that depicts a step 10. of the exemplary method for using the medical procedure shown in Fig. 6.
Fig. 17 is a perspective view that depicts a configuration of a medical device according to an embodiment of the present invention.
Fig. 18 is a front view that depicts a configuration of a main body of the medical device according to the embodiment.
Fig. 19 depicts a front view and a sectional view of a suture insertion needle of the medical device according to the embodiment.
Fig. 20 depicts a front view and a sectional view of a suture grasping needle of the medical device according to the embodiment.
Fig. 21 is a front view that depicts a suture traction tool of the medical device according to the embodiment.
Fig. 22 is a sectional front view that depicts a state in which an abutment member of the medical device according to the embodiment abuts on an abdominal wall and in which the abdominal wall is punctured perpendicularly by the suture grasping needle.
Fig. 23 is a sectional front view that depicts a state in which the suture traction tool of the medical device according to the embodiment is inserted into the suture grasping needle.
Fig. 24 is a sectional front view that depicts a state in which the suture insertion needle is inserted into an inside of an annular portion of the suture traction tool of the medical device according to the embodiment.
Fig. 25 is a sectional front view that depicts a state in which the suture protrudes from a tip end of the suture insertion needle of the medical device according to the embodiment.
Fig. 26 is a sectional front view that depicts a state in which the suture insertion needle of the medical device according to the embodiment is pulled out.
Fig. 27 is a sectional front view that depicts a state in which the main body is rotated by about 180 degrees about the suture insertion needle as a rotational axis of the medical device according to the embodiment.
Fig. 28 is a sectional front view that depicts a state in which the suture insertion needle of the medical device according to the embodiment punctures again the body and in which the suture is inserted into the needle.
Fig. 29 is a sectional front view that depicts a state in which the suture traction tool of the medical device according to the embodiment is withdrawn and the suture is grasped by the annular portion.
Fig. 30 is a sectional front view that depicts a state of inserting a guide wire of the medical device according to the embodiment.
Fig. 31 is a sectional front view that depicts a state in which sutures inserted from two positions at which the suture insertion needle of the medical device according to the embodiment punctures pass from the abdominal wall into the stomach wall, and protrudes from positions at which the suture grasping needle punctures the body.
Fig. 32 is a perspective view that depicts a configuration of a medical device according to an embodiment of the present invention.
Fig. 33 is a front view that depicts a configuration of a main body of the medical device according to the embodiment.
Fig. 34 depicts a front view and a sectional view of a suture insertion needle of the medical device according to the embodiment.

### DETAILED DESCRIPTION OF THE INVENTION

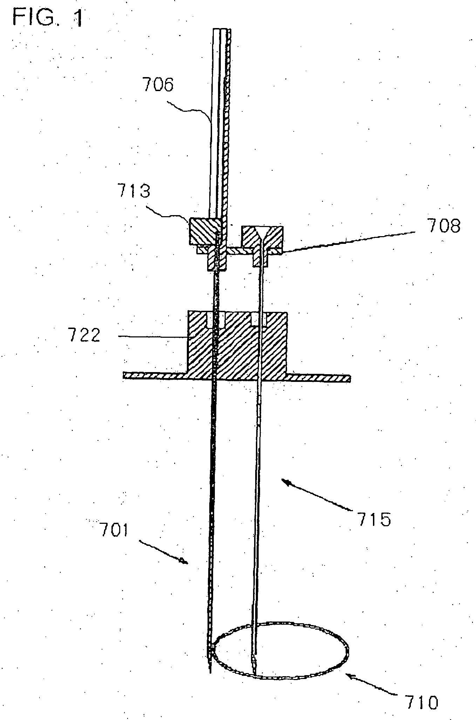

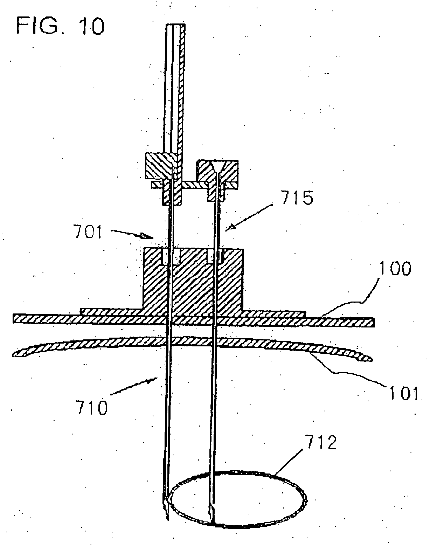

Embodiments of the present invention will be described hereinafter in detail with reference to the drawings. A medical device described hereinafter is a suture fixing tool for medical treatments used for suturing a body surface part and an internal tissue. This medical suture fixing tool is specifically used to fix an abdominal wall to a stomach wall for facilitating insertion of a catheter in percutaneous endoscopic gastrostomy conducted to, for example, supply nutritional supplements or discharge a body fluid. In Figs. 1 to 5 and Figs. 7 to 34, an upside of each sheet is a base end (rear end) side of the suture and a downside of each sheet is a tip end side of the suture.

### (FIRST EMBODIMENT)

Fig. 1 is a sectional view that depicts a configuration of a medical device according to the present embodiment. The medical device shown in Fig. 1 includes a suture grasping needle 701, a guidematerial 706, a fixing member 708, a suture traction tool 710, a suture insertion needle 715, and an abutment member 722. This medical device can be used as, for example, an abdominal wall-to-stomach wall fixing tool.

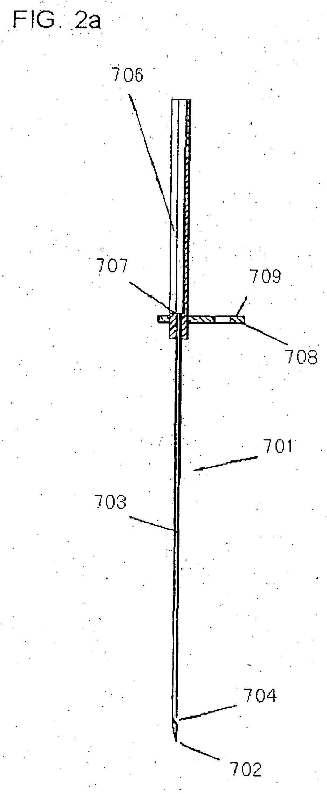

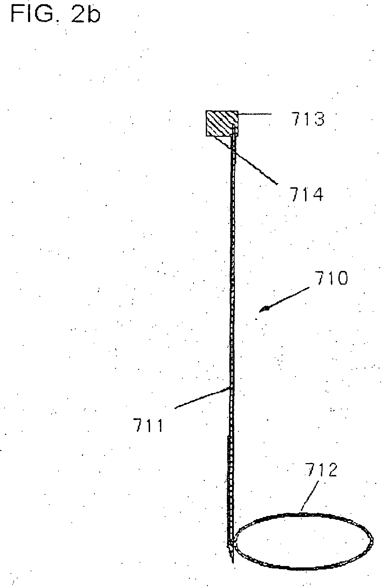

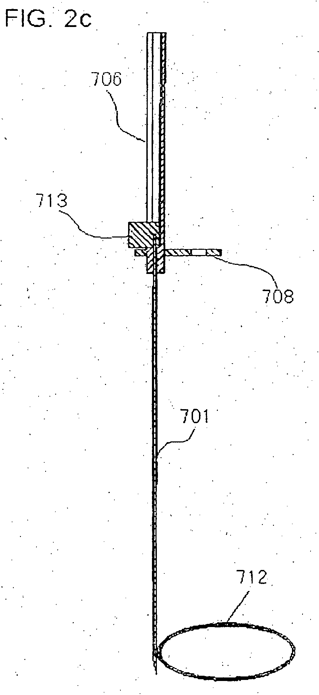

Figs. 2a to 2c depict a configuration of the suture grasping needle 701 and those of peripheral members of the puncture needle 701. Fig. 2a is a sectional view that depicts a state in which the suture grasping needle 701 is combined with the guide material 706 and the fixing member 708. Fig. 2b is a sectional view that depicts a configuration of the suture traction tool 710. Fig. 2c is a sectional view that depicts a state of combining Figs. 2a and 2b.

As shown in Figs. 2a to 2c, a rod 711 and a snare 712 of the suture traction-tool 710 are configured to be able to be stored in a hollow portion 703 of the suture grasping needle 701. By allowing a slide material 713 to slide along the guide material 706, the suture traction tool 710 is slidably moved within the hollow portion 703, and the snare 712 provided on a tip end of the suture traction tool 710 can be pulled out from a lateral hole 704 of the suture grasping needle 701 to an outside of the hollow portion 703.

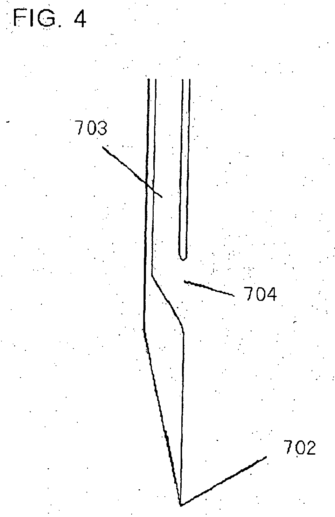

Fig. 4 is an enlarged sectional view of a tip end of the suture grasping needle 701. The suture grasping needle 701 is a hollow needle member consisting of metal and includes a sharp tip end 702. Because of the sharp tip end 702, the suture grasping needle 701 can easily puncture the skin. In addition, the suture grasping needle 701 is configured so that the hollow portion 703 formed inside does not penetrate the tip end 702. The hollow portion 703 is structured to communicate with the outside through the lateral hole 704 provided near the tip end 702. The suture traction tool 710 can be slidably moved within the hollow portion 703. Specifically, an outside diameter of the suture grasping needle 701 can be set, for example, equal to or larger than 12G and equal to or smaller than 22G, preferably equal to or larger than 19G and equal to or smaller than 21G. If a guide wire is inserted into the hollow portion 703, the outside diameter of the suture grasping needle 701 can be set, for example, equal to or larger than 16G and equal to or smaller than 18G. By so setting, it is possible to easily insert the suture traction tool and the guide wire simultaneously into the suture grasping needle 701, and appropriately insert the guide wire into a living body together with the suture.

An entire length of the suture grasping needle 701 is not limited to a specific length; however, it can be set, for example, equal to or larger than 20 mm and equal to or smaller than 200 mm, preferably equal to or larger than 80 mm and equal to or smaller than 100 mm. If the entire.length is too small, the suture grasping needle 701 may possibly be buried in an abdominal wall of a patient. If too large, operativity of the medical device may possibly be deteriorated.

In the suture grasping needle 701, a portion that may possibly contact with the suture during a suturing operation may be configured to be non-sharp. As will be described later, the lateral hole 704, in particular, possibly in contact with the suture when the suture is grasped by the suture traction tool 710, attracted and held by the suture grasping needle 701 is configured to be non-sharp. Specifically, an edge of the lateral hole 704 can be chamfered to have a C-shaped surface or an R-shaped surface.

Further, the suture grasping needle 701 can be configured so that no difference in height is present in the hollow portion 703 continuous from a rear end of the suture grasping needle 701 to the lateral hole 704. By doing so, when the suture traction tool 710 is slidably moved within the hollow portion 703, the suture traction tool 710 can smoothly slide without catching the snare 712 in the tractor 710. Specifically, in a portion of the hollow portion 703 continuous to the lateral hole 704, the hollow portion 703 can be configured so that a central axis of the lateral hole 704 is inclined from that of the suture grasping needle 701 at 120 to 150 degrees.

The guide material 706 is attached to the rear end of the suture grasping needle 701 in a direction opposite to an extension direction of the suture grasping needle 701. The guide material 706 is slidably fitted into the slide material 713 attached to a rear end of the suture traction tool 710 to be described later. A shoulder surface 707 of the guide material 706 abuts on a shoulder surface 714 of the slide material 713, whereby the suture traction tool 710 is slidably moved within the hollow portion 703 of the suture grasping needle 701, and the guide material 706 functions to indicate that the suture traction tool 710 reaches a forward limit.

Further, this guide material 706 is fitted into the slide material 713 only in one direction. Due to this, if the suture traction tool 710 is slidably moved within the hollow portion 703 of the suture grasping needle 701 and reaches the forward limit, it is possible to ensure that the snare 712 of the suture traction tool 710 is widened in a direction of the suture insertion needle 715.

The fixing member 708 is attached to the rear end of the suture grasping needle 701. The fixing member 708 is configured so that a shoulder surface 709 thereof is fitted into that of a stopper (not shown) of the suture insertion needle 715, to be described later, only in a certain direction to prevent the fixing member 708 from being relatively rotated to the puncture needle 715. By doing so, it is possible to ensure that a lateral hole (not shown)' of the suture insertion needle 715 is arranged on the suture grasping needle 701 side. In addition, by so fitting, a tip end (not shown) of the suture insertion needle 715 can be positioned only at one position forward of that of the tip end 702 of the suture grasping needle 701.

The suture traction tool 710 includes the rod 711, the snare 712 attached to a tip end of the rod 711, and the slide material 713 attached to a rear end thereof. The rod 711 can be formed out of metal. The snare 712 is formed out of an elastically deformable material.

The snare 712 is annular in a free state. If stored in the hollow portion 703 of the suture grasping needle 701, the snare 712 is linearly and elastically deformed. As a result, the snare 712 is shaped into an annular body when being forced out from the lateral hole 704 to the outside of the suture grasping needle 701. The snare 712 can, therefore, easily capture a portion of the suture (not shown) exposed from the tip end of the suture insertion needle 715.

As stated above, the slide material 713 attached to the rear end of the suture traction tool 710 is slidably fitted into the guide material 706 attached to the rear end of the suture grasping needle 701 to abut the shoulder surface 714 of the slide material 713 on the shoulder surface 707 'of the guide material 706. Thus, the slide material 713 functions to indicate that the suture traction tool 710 reaches the forward limit within the hollow portion 703 of the suture grasping needle 701.

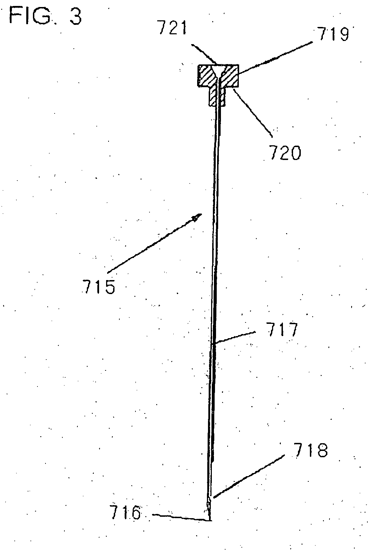

Fig. 3 is a sectional view that depicts a configuration of the suture insertion needle 715. A shape of the suture insertion needle 715 can be designed similar to that of the suture grasping needle 701 shown in Fig. 4. The suture insertion needle 715 is a hollow needle consisting of metal and includes a sharp tip end 716. Because of the sharp tip end 716, the suture insertion needle 715 can easily puncture the skin. In addition, a hollow portion 717 formed inside the suture insertion needle 715 is configured not to penetrate the tip end 716. The hollow portion 717 is structured to communicate with the outside through the lateral' hole 718 provided near the tip end 716. The suture can be inserted into this hollow portion 717.

Specifically, an outside diameter of the suture insertion needle 715 can be set, for example, equal to or larger than 15G and equal to or smaller than 25G, preferably equal to or larger than 19G and equal to or smaller than 21G. By so setting, it is possible to reduce a puncture resistance of the suture insertion needle 715 and the suture insertion needle 15 can easily puncture the skin.

An entire length of the suture insertion needle 715 is not limited to a specific length; however, it can be set, for example, equal to or larger than 20 mm and equal to or smaller than 200 mm, preferably equal to or larger than 80 mm and equal to or smaller than 100 mm. If the entire length is too small, the suture insertion needle 715 may possibly be buried in the abdominal wall of the patient. If too large, the operativity of the medical device may possibly be deteriorated.

In the suture insertion needle 715, a portion that may possibly contact with the suture can be configured to be non-sharp. The lateral hole 718 of the suture insertion needle 715, in particular, possibly in contact with the suture when the suture is grasped by the suture traction tool 710 and attracted to the suture grasping needle 701 is configured to be non-sharp. Specifically, an edge of the lateral hole 718 can be chamfered to have a C-shaped surface or an R-shaped surface.

Further, it is preferable that no difference in height is present in'the hollow portion 717 continuous from the rear end of the suture insertion needle 715 to the lateral hole 718 so that the suture can be smoothly inserted into the hollow portion 717. Specifically, in a portion of the hollow portion 717 continuous to the lateral hole 718, the hollow portion 717 can be configured so that a central axis of the lateral hole 718 is inclined from that of the suture insertion needle 715 at 120 to 150 degrees.

A stopper 719 is attached to the rear end of the suture insertion needle 715. As stated, a shoulder surface 720 of this stopper 719 is fitted into the shoulder surface 709 of the fixing member 708 attached to the rear end of the suture grasping needle 701 only in one direction so as to prevent from being relatively rotated. It is thereby possible to ensure that the lateral hole 718 of the suture insertion needle 715 is oriented to the direction of the suture grasping needle 701. By thus fitting, the tip end 716 of the suture insertion needle 715 can be positioned only at one position forward of that of the tip end 702 of the suture grasping needle 701.

A tapered hole 721 for inserting the suture is formed in the stopper 719. A diameter of a large-diameter side of this tapered hole 721 is larger than an inside diameter of the suture insertion needle 715. The diameter of the tapered hole 721 is smaller as nearer to the suture insertion needle 715, and a diameter of a small-diameter side of the tapered hole 721 is substantially equal to the inside diameter of the suture insertion needle 715. The configuration in which no difference in height is present between this tapered hole 721 and the inside of the suture insertion needle 715 can be thereby provided. Due to this, the suture (not shown) can be smoothly inserted into the suture insertion needle 715 from the rear end thereof.

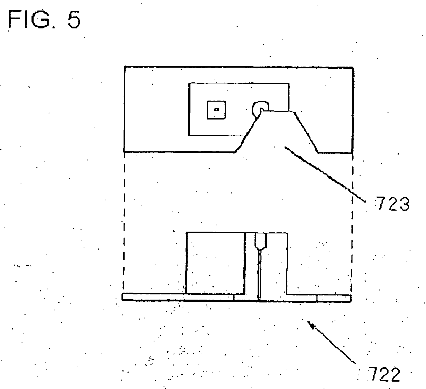

Fig. 5 depicts a front view and a plan view of the abutment member 722. The abutment member 722 keeps a distance between the suture grasping needle 701 and the suture insertion needle 715 constant and holds the needles 701 and 715 in parallel to each other. As will be described later, the abutment member 722 abuts on suture targets such as a body surface part during the suturing operation. The medical device can be, therefore, held on each suture target during the suturing operation. The suture targets can be, therefore, surely put in the suture. The suture grasping needle 701 and the suture insertion needle 715 can be slidably moved within the abutment member 722 independently of each other.

A notch 723 is formed on a side of the abutment member 722 on which side the suture insertion needle 715 is located. A width of a portion of this notch 723 which portion contacts with the suture insertion needle 715 is set, for example, about φ0.25 to 0.5 mm. By so setting, while the suture insertion needle 715 is removed from the abutment member 722, the suture 705 from the notch 723 can be removed and the abutment member 722 can be easily rotated about the suture grasping needle 701. In addition, by thus configuring, as will be described later, a plurality of puncture positions of the suture insertion needle 715 can be arranged concentrically about the puncture position of the suture grasping needle 701 set as one end of the planned suture site.

As a material for this abutment member 722, a high transparency synthetic resin material, for example, can be used. The abutment member 722 is disposed on a site in which a small incision is made in the abdomen with a knife. If the material for the abutment member 722 is a transparent material, it is possible to further ensure performing the suturing operation.

As can be understood, according to this embodiment, the suture insertion needle 715 and the suture grasping needle 701 each exhibiting a high puncture performance, and the abutment member 722 capable of improving user-friendliness without simultaneously allowing these puncture needles 715 and 701 to puncture the target site can be provided. In addition, the medical device capable of concentrically arranging a plurality of suture sites can be stably provided. It is, therefore, possible to improve the performance of the medical device for suturing the abdominal wall and the stomach wall by the suture in the percutaneous endoscopic gastrostomy.

The practical exemplary method for using the medical device according to the present invention will next be described so as to clarify advantages of the claimed medical device.

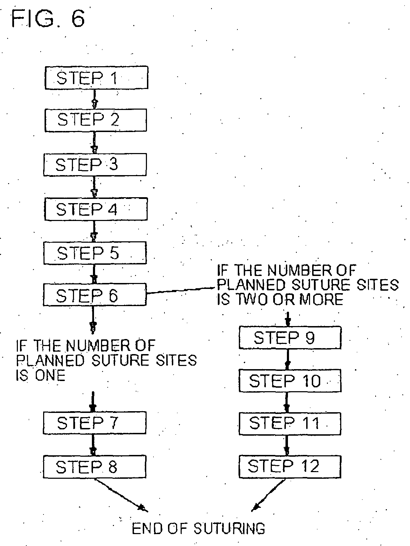

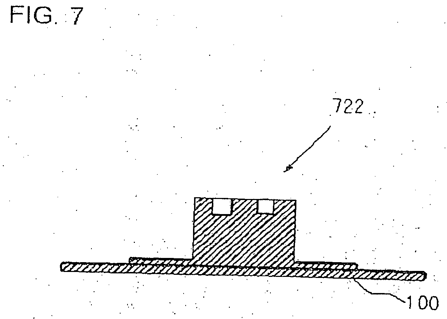

Fig. 6 is a flowchart of the exemplary method for using the medical device according to this-embodiment. Figs. 7 to 16 are explanatory' views for respective procedures in steps 1 to 10 shown in Fig. 6.

The medical device according to this embodiment is used to suture a plurality of planned suture sites separated from one another in the living body by a suture. Before using the medical device according to this embodiment, an endoscope is inserted into the stomach of the patient to sufficiently supply the air thereto, and an inner side of the abdomen is closely attached to an outer wall of the stomach. Next, a position of the stomach is confirmed by a transparent light from the endoscope, and the abdominal skin is disinfected and anesthetized locally. A small incision is made in the position of the stomach by the knife.

In a step 1, the abutment member 722 according to the present invention is disposed on the small-incision site of an abdominal wall 100 (Fig. 7).

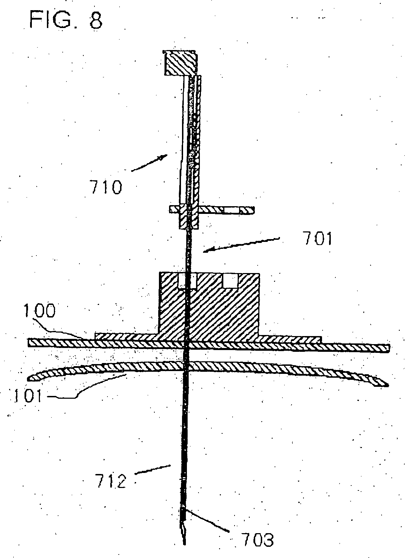

In a step 2, the suture grasping needle 701 according to the present invention punctures the abdominal wall 100 and a stomach wall 101 of the patient perpendicularly through the abutment member 722 (Fig. 8). At this time, the suture traction tool 710 is already stored in the hollow portion 703 of the suture grasping needle 701, and the snare 712 is linearly and elastically deformed.

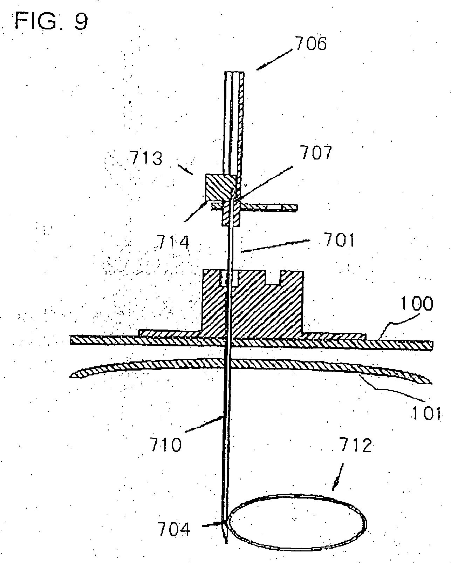

Next, in a step 3, the shoulder surface 714 of the slide member 713 attached to the rear end of the suture grasping needle 701 is allowed to slide until abutting on the shoulder surface 707 of the guide material 706 of the suture grasping needle 701 (Fig. 9). By doing so, the snare 712 of the suture traction tool 710 protrudes from the lateral hole 704 in a direction in which the suture grasping needle 701 can puncture the target site, thereby forming the snare 712 into an annular shape in a free state.

Then, in a step 4, similarly to the suture grasping needle 701, the suture insertion needle 715 punctures the abdominal wall 100 and the stomach wall 101 of the patient perpendicularly through the abutment member 722 (Fig: 10). At this time, it is confirmed by the endoscope that the suture insertion needle 715 is passed through an inside of the annular snare 712 of the suture traction tool 710.

In this embodiment, the suture grasping needle 701 and the suture insertion needle 715 are puncture needles each having the non-hollow sharp tip end. Due to this, as compared with the use of a puncture needle having a difference in height such as a hollow needle, a cannula, or an outer cylinder, the high puncture performance is ensured and the living body can be easily punctured. Further, in this embodiment, the suture grasping needle 701 and the suture insertion needle 715 are operated individually. Due to this, as compared with the instance in which the living body is punctured simultaneously by.the suture grasping needle 701 and the suture insertion needle 715 or the instance in which the suture grasping needle 701 and the suture insertion needle 715 are simultaneously operated, the medical device does not require strong force, experience, and expertise and can easily fulfill the.function of puncturing the living body.

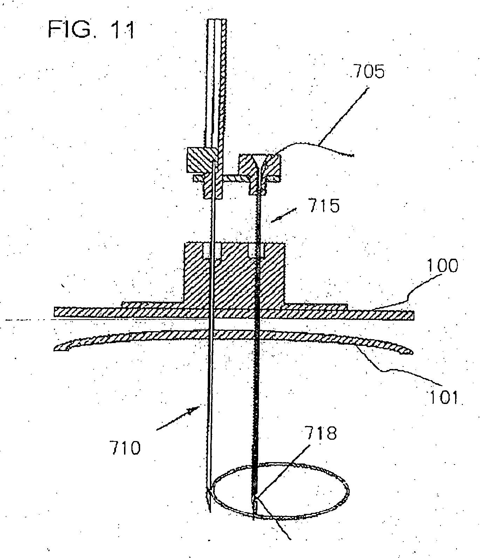

In a step 5, the suture 705 is inserted into the suture insertion needle 715 from the rear end thereof, and the end of the suture 705 is exposed from the lateral hole 718 of the suture insertion needle 715 (Fig. 11). In this state, since the suture insertion needle 715 itself is passed through the inside of the snare 712 formed into the annular shape of the suture traction tool 710, the suture 705 pulled out from the suture insertion needle 715 is also passed through the inside of the snare 712.

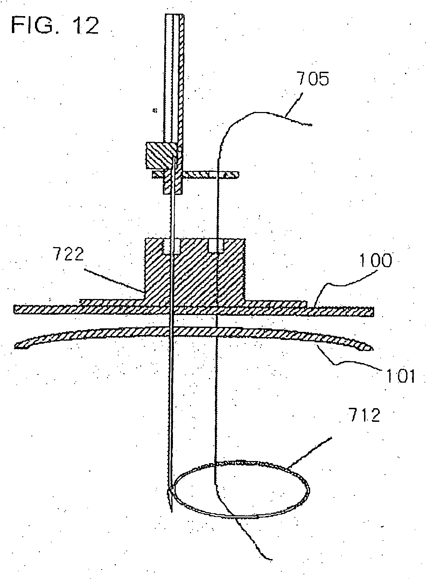

In a step 6, only the suture insertion needle 715 is pulled out from the living body and the abutment member 722 while leaving the suture 705 as it is (Fig. 12). As a result, the suture 705 is passed through the inside of the snare 712. At this time, the suture 705 may be captured once by the snare 712.

At this moment, if an operator judges that the suturing operation is finished at only one planned suture'site, the processing goes to a step 7. If the puncture position of the suture grasping needle 701 is set as one end of the planned suture site and a plurality of puncture positions of the suture insertion needle 715 are arranged concentrically around the puncture position of the suture grasping needle 701, the processing goes to a step 9.

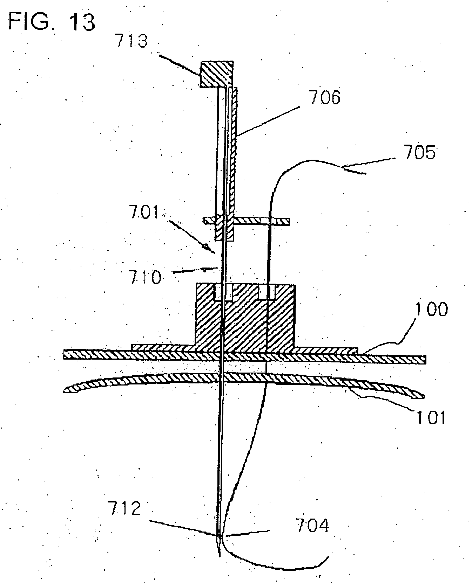

If the number of planned suture site is one and the suturing operation is finished at the one site, then the slide material 713 of the suture traction tool 710 is allowed to slide upward along the guide material 706 of the suture grasping needle 701, and the snare 712 of the suture traction tool 710 is inserted into the hollow portion 703 through the lateral hole 704 of the suture grasping needle 701 in the step 7 (Fig. 13). Accordingly, the snare 712 formed into the annular shape is elastically deformed and gradually linearized, so that the suture 705 is grasped by the snare 712 in the portion of the lateral hole 704 of the suture grasping needle 701.

In this state, the edge of the lateral hole 704 is chamfered to have the C-shaped surface or R-shaped surface. Thanks to this, it is possible to prevent the suture 705 from being cut off due to the contact between the suture 705 grasped by the snare 712 and the lateral hole 704 of the suture grasping needle 701 by excessively strong force.

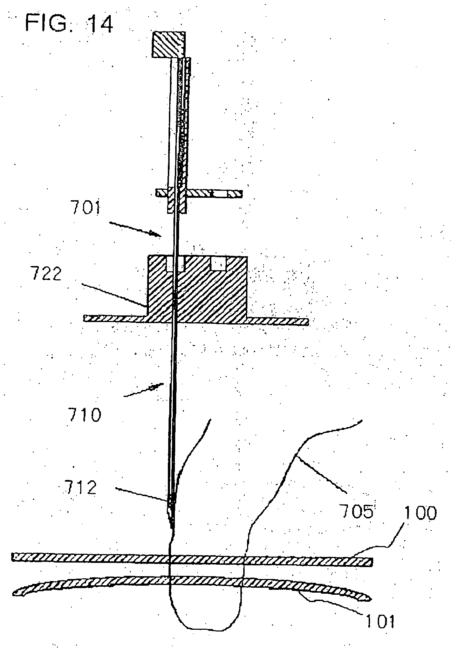

In a step 8, the suture grasping needle 701 and the abutment member 722 including the suture traction tool 710 are pulled in a direction away from the abdomen of the patient (Fig. 14). Accordingly, the suture 705 grasped by the snare 712 of the suture traction tool 710 is pulled out from the body. As a result, the suture 705 invades the body from the puncture position of the suture insertion needle 715, passes into the stomach, and is exposed to the outside of the body from the puncture position of the suture grasping needle 701.

Thereafter, using a knotter, both ends of the exposed suture are knotted together, whereby the stomach wall 101 is fixed to the abdominal wall 100 by the suture 705.

Further, if the puncture position of the suture grasping needle 701 is set as one end of the planned suture site and a plurality of puncture positions of the suture insertion needle 715 are concentrically arranged around the puncture position of the suture grasping needle 701, the processing goes from the step 5 to the step 9.

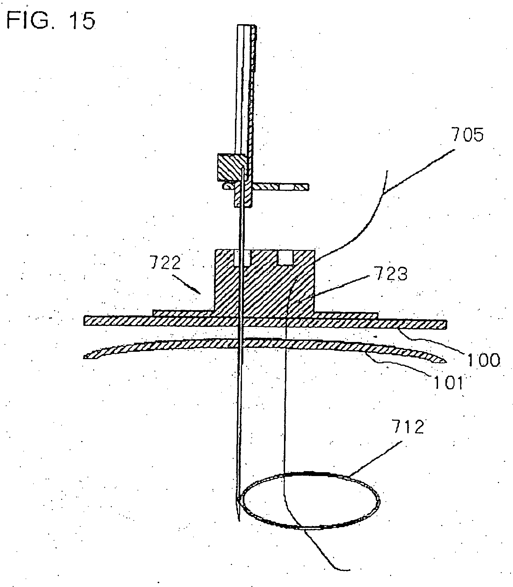

In the step 9, the suture 705 is removed from the abutment member 722 by the notch 723 of the abutment member 722 outside the body while keeping a state in which the suture 705 is passed through the inside of the snare 712 in the body (Fig. 15). Since the notch 723 is provided in the abutment member 722, it is possible to ensure removing the suture 705 from the abutment member 722 while the suture grasping needle 701 is fixed to the abutment member 722.

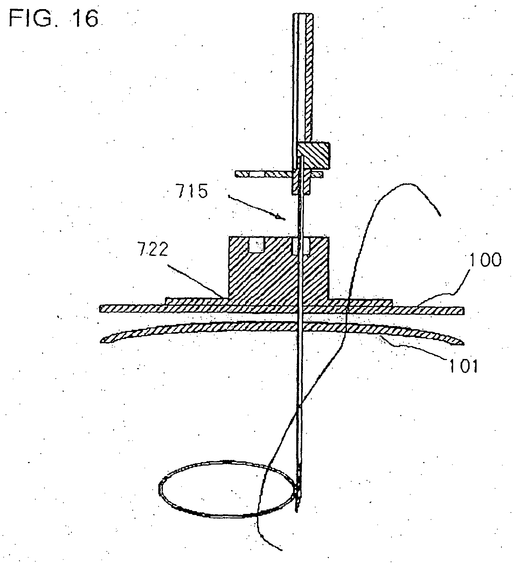

Then, in a step 10, the abutment member 722 is rotated about the puncture position of the suture grasping needle 701 in a direction of the next planned puncture position of the suture insertion needle 715 (Fig. 16). The abutment member 722 can be rotated by 360 degrees.

Next, in a step 11, similarly to the instance in which the suturing operation is finished at only one planned suture site, the steps 4 to 6 in which the suture insertion needle 715 perpendicularly punctures the abdominal wall 100 and the stomach wall 101 of the patient through the abutment member 722 are repeatedly executed by as much as the number of times of planned suturing operations.

Finally, in a step 12, similarly to the instance in which the suturing operation is finished at only one planned suture site, the step 7 in which the suture 705 is grasped by the snare 712 in the lateral hole 704 of the suture grasping needle 701 and the step 8 are executed.

By doing so, the concentric arrangement of a plurality of puncture positions of the suture insertion needle 715 around the puncture position of the puncture needle 701 which is set as one end of the planned suture site can be realized. The stomach wall 101 is fixed to the abdominal wall 100 by a plurality of sutures 705.

By rotating the abutment member 722 while the suture grasping needle 701 punctures the body, the puncture position of the suture insertion needle 715 can be adjusted. It is, therefore, possible to ensure that the stomach wall 101 is fixed to the abdominal wall 100 at a plurality of positions on a circular arc about the suture grasping needle 701 by a simple operation.

The exemplary procedures for the suturing operation using the medical device according to this embodiment are thus finished.

In this exemplary procedure, the puncture needles each having the non-hollow sharp tip end are used as the suture grasping needle 701 and the suture insertion needle 715, respectively. Due to this, as compared with the use of the puncture needle having a difference in height such as the hollow needle, the cannula or the outer cylinder, the high puncture performance is ensured and the living body can be easily punctured.

Further, the single suture grasping needle 701 and the single suture insertion needle 715 are operated individually. Due to this, as compared with the instance in which the living body is punctured simultaneously by the suture.grasping needle 701 and the suture insertion needle 715 or the instance in which the suture grasping needle 701 and the suture insertion needle 715 are simultaneously operated, the medical device does not require strong force, experience, and expertise and can lessen the stress of the patient and shorten operation time.

Furthermore, the puncture position of the suture grasping needle 701 is set as one end of the planned puncture site and a plurality of puncture positions of the suture insertion needle 715 can be arranged concentrically around the puncture position of the suture grasping needle 705. It is, therefore, possible to safely and surely fix the planned suture sites. Further, the invasion of the suture into the patient accompanying this fixing occurs quite infrequently and the patient is less burdened. It is, therefore, possible to return the patient to ordinary life at earlier time and to reduce medical cost of the patient.

A medical device according to the following embodiment is configured to employ a suture insertion needle (first puncture needle) 3 and a suture grasping needle (second puncture needle) 4.

### (SECOND EMBODIMENT)

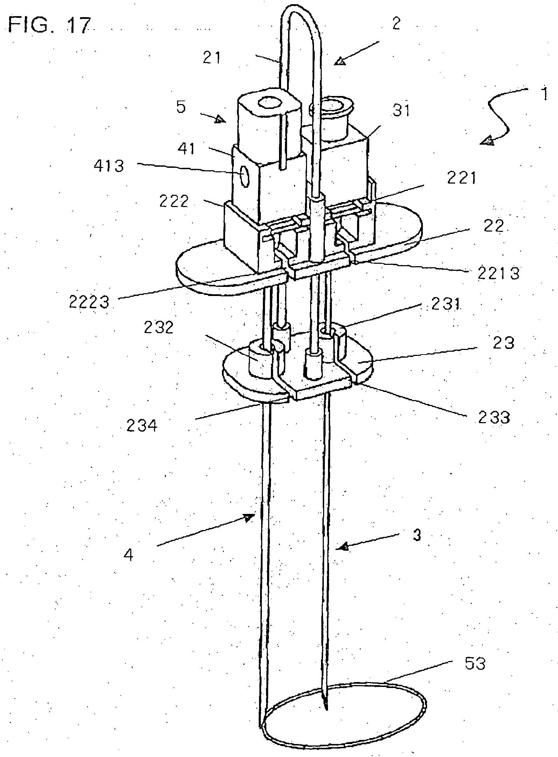

Fig. 17 is a perspective view that depicts a configuration of a medical device according to the present embodiment.

A medical device 1 shown in Fig. 17 includes a main body 2, a suture insertion needle 3 into which a suture can be inserted, and a suture grasping needle 4 which is provided to be proximate to the suture insertion needle 3 and into which a suture traction tool can be inserted. The suture insertion needle 3 and the suture grasping needle 4 are detachably attached to the main body 2 by attachment and detachment means.

In this embodiment and a third embodiment, the suture insertion needle 3 corresponds to the suture insertion needle 715 (shown in Fig. 1) of the medical device according to the first embodiment. The suture grasping needle 4 corresponds to the suture grasping needle 701 (shown in Fig. 1) of the medical device according to the first embodiment.

The respective constituent elements of the medical device 1 will be described.

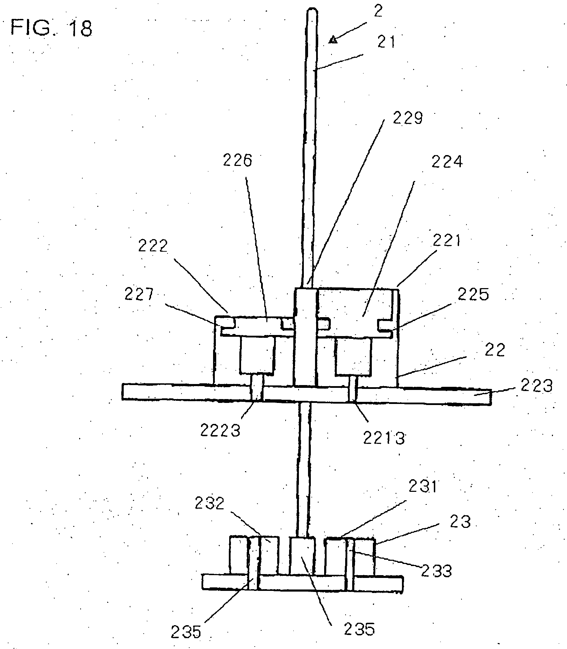

Fig. 18 is a front view that depicts a configuration of the main body 2 of the medical device 1. The main body 2 has a function of supporting the suture insertion needle 3 and the suture grasping needle 4.

The main body 2 has a support member 21, a fixing member 22 provided on a base end of the support member 21 (an upper side thereof in Fig. 17), and an abutment member 23 provided on a tip end thereof (a lower side thereof in Fig. 17).

In this embodiment and the third embodiment, the abutment member 23 corresponds.to the abutment member 722 (shown in Fig. 1) of the medical device according to the first embodiment. The fixing member 22 corresponds to the fixing member 708 (shown in Fig. 1) of the medical device according to the first embodiment.

The support member 21, which is U-shaped, supports the fixing member 22 and the abutment member 23. The suture insertion needle 3 and the suture grasping needle 4 are supported by the support member 21 through the abutment member 23. Examples of materials for the support member 21 include vinyl chloride resin, polycarbonate resin, ABS resin, polyacetal resin, polyamide resin, polypropylene resin, polyethylene resin or the like, and metal such as stainless steel.

The fixing member 22 includes a function of fixing the suture insertion needle 3 and the suture grasping needle 4. As shown in Fig. 18, the fixing member 22 includes a first fixing portion 221 and a second fixing portion 222 that fix the suture insertion needle 3 and the suture grasping needle 4, respectively, and a plate body 223. The first fixing portion 221, the second fixing portion 222, and the plate body 223 are formed integrally.

The first fixing portion 221 and the second fixing portion 222 are provided on a base end-side surface (an upper side in Fig. 18) of the plate body 223. The first fixing portion 221 and the second fixing portion 222 are provided to be symmetric about the support member 21.

The first fixing portion 221 and the second fixing portion 222 have about the same structure and generally rectangular parallelepiped. The first fixing portion 221 is higher than the second fixing portion 222. It is thereby possible for a user to easily visually distinguish the first fixing portion 221 from the second fixing portion 222. In this embodiment, the first fixing portion 221 differs in height from the second fixing portion 222. Alternatively, the first fixing portion 221 and the second fixing portion 222 may be equal in height.

A first concave portion 224 is provided in the first fixing portion 221 into which the suture insertion needle 3 can be inserted.

In the first concave portion 224, a first flange engagement portion 225 engageable with a first flange of a first hub 31 (not shown in Figs. 17 and 18), to be described later, provided on the suture insertion needle 3 is formed.

The first concave portion 224 is hollowed out cylindrically, smaller in diameter on a tip end and larger on a base end across the first flange engagement portion 225.

The first concave portion 224 is opened to a front surface side of the first fixing portion 221, so that the first hub 31 of the suture insertion needle 3, to be described later, can be inserted from the front surface'side thereof.

The first flange (not shown in Figs. 17 and 18) can be' engaged with the first flange engagement portion 225. While the first flange (not shown in Figs. 17 and 18) is engaged with the first flange engagement portion 225, the suture insertion needle 3 cannot either rotate or move vertically.

Further, the suture insertion needle 3 can be inserted into the first concave portion 224 from the base end side without engaging the first flange (not shown in Figs. 17 and 18) with the first flange engagement portion 225. In this case, similarly to the above, an outer circumference of the first flange (not shown in Figs. 17 and 18) is fitted into the first concave portion 224 and fixed thereto, so that the suture insertion needle 3 cannot either rotate or move vertically.

The second fixing portion 222 includes a second concave portion 226 into which the suture grasping needle 4 can be inserted.

In the second concave portion 226, a second flange engagement portion 227 engageable with a second flange of a second hub 41 (not shown in Figs. 17 and 18), to be described later, provided on the suture grasping needle 4 is formed.

The second concave portion 226 is hollowed out cylindrically, smaller in diameter on a tip end and larger on a base end across the second flange engagement portion 227.

The second concave portion 22 6 is opened to a front surface side of the second fixing portion 222, so that the second hub 41 of the suture grasping needle 4, to be described later, can be inserted from the front surface side thereof.

A first notch 2213 and a second notch 2223 are formed in the first fixing portion 221 and the second fixing portion. 222 so that the suture insertion needle 3 and the suture grasping needle 4 are detachable, respectively. It is thereby possible to make the suture insertion needle 3 and the suture grasping needle 4 detachable, so that operativity can be improved and it is possible to ensure performing suturing by a simple operation.

The first notch 2213 and the second notch 2223 are formed from a front edge of the fixing member 22 deep into the first concave potion 224 and the second concave portion 227 through the first fixing portion 221 and the second fixing portion 222, respectively.

The fixing member 22 includes a slide portion 229 that allows the fixing member 22 to be slidably moved relative to the support member 21. The fixing member 22 can be thereby freely moved forward or backward relative to the support member 21, thereby facilitating puncturing by the suture insertion needle 3 and the suture grasping needle 4.

As a material for the fixing member 22, a high transparency synthetic resin material, for example, is preferably used since the fixing member 22 is disposed on a site in which a small incision is made in the body surface part such as the abdomen with a knife.

The abutment member 23 functions to improve stability when the medical device 1 is used by causing the abutment member 23 to abut on the body surface part.

As shown in Figs. 17 and 18, the abutment member 23 is flat, and includes a first cylindrical portion 231 that includes an inner cavity into which the suture insertion needle 3 can be inserted, and a second cylindrical portion 232 that includes an inner cavity into which the suture grasping needle 4 can be inserted, on a base end-side surface thereof.

The first cylindrical portion 231 and the second cylindrical portion 232 function to improve stability on the puncture by the suture insertion needle 3 and the suture grasping needle 4, respectively.

The abutment member 23 includes a first notch 233 and a second notch 234 serving as the attachment and detachment means so that the suture insertion needle 3 and the suture grasping needle 4 are detachable to the abutment member 23, respectively. The abutment member 23 can thereby detachably hold the suture insertion needle 3 and the suture grasping needle 4, thus improving the operativity.

The first notch 233 and the second notch 234 are formed from bottom portions of the abutment member 23 deep into the first cylindrical portion 231 and the second cylindrical portion 232 therethrough, respectively. The suture insertion needle 3 and the suture grasping needle 4 are thereby made detachable to the abutment member 23.

Further, the abutment member 23 includes two convex portions 235 having a concave portion at a center thereof into which one end of the support member 21 can be inserted. One end of the support member 21 is inserted into the concave portion of the convex portions 235.

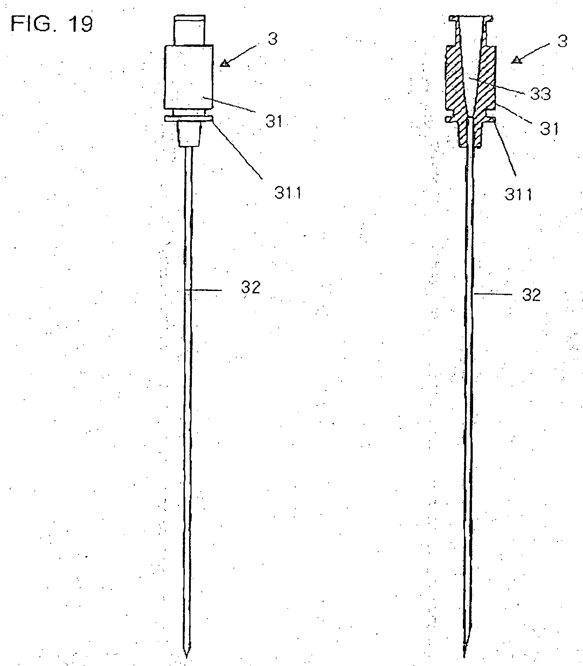

Fig. 19 depicts a front view and a sectional view of the suture insertion needle 3. The suture insertion needle 3 functions to insert the suture into an internal tissue.

As shown in Fig. 19, the suture insertion needle 3 includes the first hub 31 provided on a base end thereof (upper side in Fig. 19) and a first needle portion 32 provided on a tip end thereof. The first hub 31 and the first needle portion 32 include a hollow portion 33, which serves as a passage of the suture.

In this embodiment and the third embodiment, the first hub 31 corresponds to the stopper 719 (Fig. 3) of the medical device according to the first embodiment.

The first hub 31 is generally cylindrical and includes a first flange 311 on a tip end side thereof, engageable with the first flange engagement portion 225. The hollow portion 33 within the first hub 31 is formed so that an inside diameter is gradually smaller from a base end to a tip end.

The first needle portion 32 includes an opening on a tip end thereof (lower side in Fig. 19) to communicate with the hollow portion 33. The first needle portion 32 keeps a constant outside diameter from a base end near to a tip end and has a sharp needle point formed on the tip end.

In this embodiment and the third embodiment, a shape of the first needle portion 32 can be designed, for example, as that of the suture insertion needle 715 (Fig. 4) according to the first embodiment. By so designing, in the first needle portion 32, it is possible to ensure that a lateral hole communicating with the outside is oriented to a second needle portion 42. In addition, the suture can be smoothly inserted.

An outside diameter of the first needle portion 32 is not limited to a specific diameter; however, it can be set, for example, equal to or larger than 15G and equal to or smaller than 25G, preferably equal to or larger than 19G and equal to or smaller than 21G. By so setting, it is possible to reduce a puncture resistance of the suture insertion needle 3 and the suture insertion needle 3 can easily puncture the body.

An entire length of the first needle portion 32 is not limited to a specific length; however, it can be set, for example, equal to or larger than 20 mm and equal to or smaller than 200 mm, preferably equal to or larger than 80 mm and equal to or smaller than 100 mm. If the entire length is too small, the first needle portion 32 may possibly be buried in the abdominal wall of the patient. If too large, the operativity of the medical device may possibly be deteriorated.

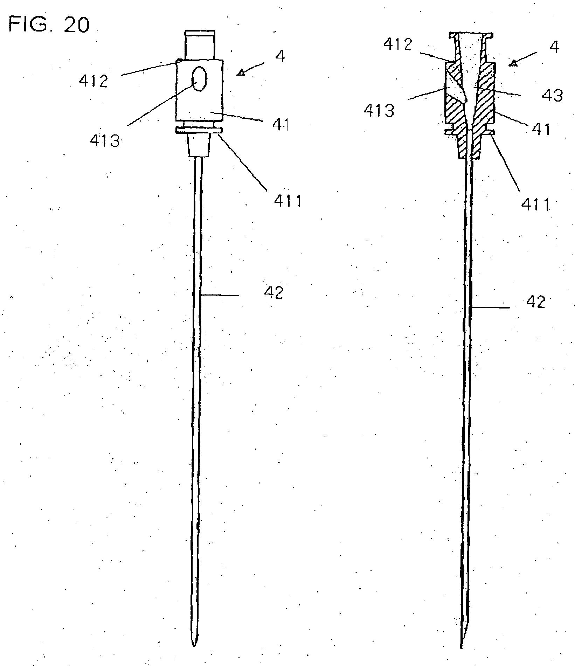

Fig. 20 depicts a front view and a sectional view of the suture grasping needle 4. The suture grasping needle 4 functions to insert the suture traction tool into the internal tissue. Further, by forming a space into which a guide wire can be inserted, in the puncture needle 4, the guide wire as well as the suture can be inserted into the living body. A position at which the guide wire is inserted can act as a catheter insertion position.

As shown in Fig. 20, the suture grasping needle 4 includes the second hub 41 provided on a base end thereof (upper side in Fig. 20) and the second needle portion 42 provided on a tip end thereof. The second hub 41 and the second needle portion 42 include a hollow portion 43, which serves as a passage of the suture traction tool, and further serves as a passage of a guide wire to be described later.

The second hub 41 is generally cylindrical, and includes a second flange 411 on a tip end thereof, engageable with the second flange engagement portion 227 and an abutment portion 412 which is provided on a base end thereof and on which a handle portion 51 of the suture traction tool 5 abuts. The hollow portion 43 within the second hub 41 is formed so that an inside diameter is gradually smaller from a base end to a tip end.

A side port 413 into which the guide wire can be inserted is privided on a side portion of the second hub 41. An outside diameterof the guide wire is not limited to a specific diameter; however, it can be set, for example, equal to or larger than 0.1 mm and equal to or smaller than 0.8 mm, preferably equal to or larger than 0.4 mm and equal to or smaller than 0.6 mm. If the outside diameter is too small, the guide wire is less firm and operativity of the guide wire may possibly be deteriorated. If too large, the guide wire may possibly damage the inside of the stomach.

The second needle portion 42 includes an opening on a tip end thereof (lower side in Fig. 20) to communicate with the hollow portion 43.

The second needle portion 42 keeps a constant outside diameter from a base end near to a tip end and has a fine edge formed on the tip end. A shape of the second needle portion 42 can be designed, for example, as that of the suture grasping needle 701 according to the first embodiment (shown in Fig. 4). By so designing, in the second needle portion 42, it is possible to ensure that a lateral hole communicating with the outside is oriented to the first needle portion 32. In addition, it is possible to allow the suture traction tool and the guide wire to smoothly slide from the hollow portion 43 to an outside of the second needle portion 42.

An outside diameter of the second needle portion 42 is not limited to a specific diameter; however, it can be set, for example, equal to or larger than 12G and equal to or smaller than 22G, preferably equal to or larger than 16G and equal to or smaller than 18G. By so setting, it is possible to easily simultaneously insert the suture traction tool and the guide wire into the suture grasping needle 4, and the guide wire as well as the suture can easily puncture the body.

An entire length of the second needle portion 42 is not limited to a specific length; however, it can be set, for example, equal to or larger than 20 mm and equal to or smaller than 200 mm, preferably equal to or larger than 80 mm and equal to or smaller than 100 mm. If the entire length is too small, it may possibly be buried in the abdominal wall of the patient. I f too large, the operativity of the medical device may possibly be deteriorated.

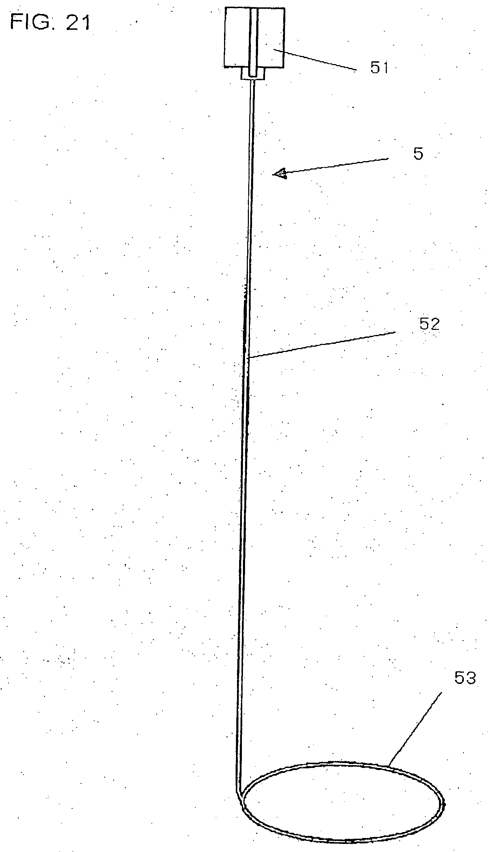

Fig. 21 is a front view that depicts a configuration of the suture traction tool 5. As shown in Fig. 21, the suture traction tool 5 includes a rod portion 52, the handle portion 51 provided on a base end (upper side in Fig. 21) of the rod portion 52, and an annular portion 53 provided on a tip end thereof. The suture traction tool 5 functions to grasp the suture inserted from the suture insertion needle 3 in the internal tissue, and to tract the suture up to the body surface part. It is thereby possible to easily put the body surface portion and the internal tissue in the suture.

The suture traction tool 5 according to this embodiment and the third embodiment corresponds to the suture traction tool 710 (shown in Fig. 1) of the medical device according to the first embodiment. The annular portion 53 corresponds to the snare 712 (shown in Fig. 2b) of the medical device according to the first embodiment.

The handle portion 51 is generally rectangular parallelepiped and an inside surface thereof is hollowed out generally cylindrically. The handle portion 51 is designed to be able to abut on a base end surface of the abutment portion 412 of the second hub 41 of the suture grasping needle 4.

An outside diameter of the rod portion 52 is desired to be smaller than an inside diameter of the suture grasping needle 4. The outside diameter of the rod portion 52 is not limited to a specific diameter; however, it can be set, for example, equal to or larger than 0.3 mm and equal to or smaller than 0.8 mm, more preferably equal to or larger than 0.5 mm and equal to or smaller than 0.6 mm. If the outside diameter is too small, strength of the suture traction tool 5 sometimes cannot be sufficiently secured. If too-large, it is often difficult to insert the guide wire.

A length of the rod portion 52 is not limited to a specific length; however, it can be set, for example, equal to or larger than 45 mm and equal to or smaller than 225 mm, preferably equal to or larger than 105 mm and equal to or smaller than 125 mm. If the length is too small, the annular portion 53 may possible not be able to sufficiently protrude from the tip end (lower side in Fig. 20) of the needle portion 42 of the suture grasping needle 4, and the annular portion 53 often may possible not be able to grasp the suture. If too large, the suture pulled out from the tip end (lower side in Fig. 19) of the first needle portion 32 of the suture insertion needle 3 may possibly not be able to be surely inserted into the annular portion 53 of the suture traction tool 5.

The operativity of the suture grasping needle 4 when the suture traction tool 5 and the guide wire are simultaneously inserted into the suture grasping needle 4 depends on a clearance (a gap distance) between the outside diameter of the rod portion 52 or that of the guide wire and the inside diameter of the suture grasping needle 4. The combination of the inside diameter of the suture grasping needle 4, and the outside diameter of the rod portion 52 of the suture traction tool 5, and the outside diameter of the guide wire can be set so that the clearance is, for example, equal to or larger than 0.05 mm and equal to or smaller than 0.30 mm. By doing so, the operativity can be surely improved.

The annular portion 53 is deformed and substantially linearized within the suture grasping needle 4, and annular when protruding from the suture grasping needle 4. This can facilitate grasping the suture. A material for the annular portion 53 is, therefore, preferably an elastic material as will be described later.

As shown in Figs. 17 and 21, the medical device is formed so that a central axis of the suture insertion needle 3 or an extension thereof penetrates the inside of the annular portion 53 when the annular portion 53 protrudes from the suture grasping needle 4. It is thereby possible to ensure grasping the suture.

A diameter of the annular portion 53 is not limited to a specific diameter; however, it can be set, for example, equal to or larger than 1.5L and equal to or smaller than 3.0L (mm) if the distance between the suture insertion needle 3 and the suture grasping needle 4 is L (mm). By so setting, it is possible to further ensure grasping the suture.

A line diameter of the annular portion 53 is not limited to a specific one; however, it can be set, for example, equal to or larger than 0.1 mm and equal to or smaller than 0.3 mm, preferably equal to or larger than 0.14 mm and equal to or smaller than 0.24 mm. If the line diameter is too small, it is sometimes difficult for the annular portion 53 to be deformed into a linear state and an annular state when a high viscosity body fluid adheres to the annular portion 53. If the line diameter is too large, the suture traction tool 5 and the guide wire may possibly not be able to be simultaneously inserted into the suture grasping needle 4.

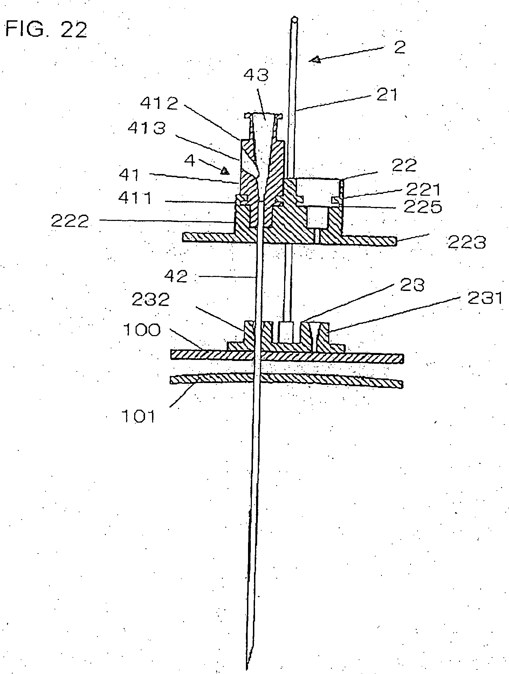

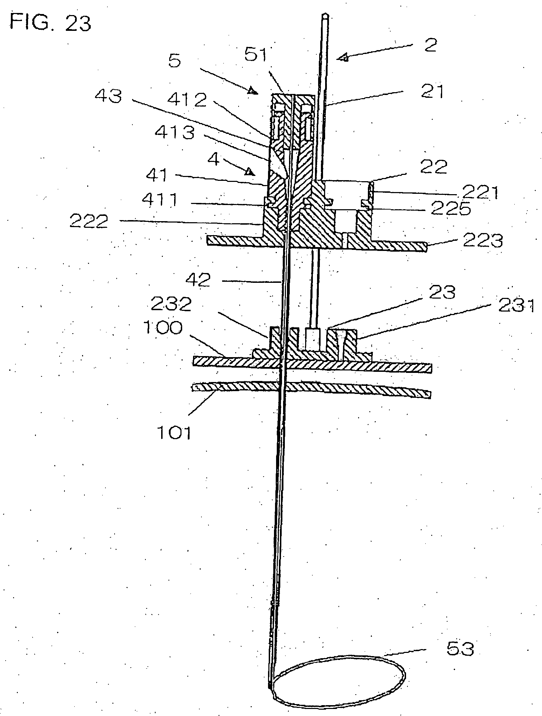

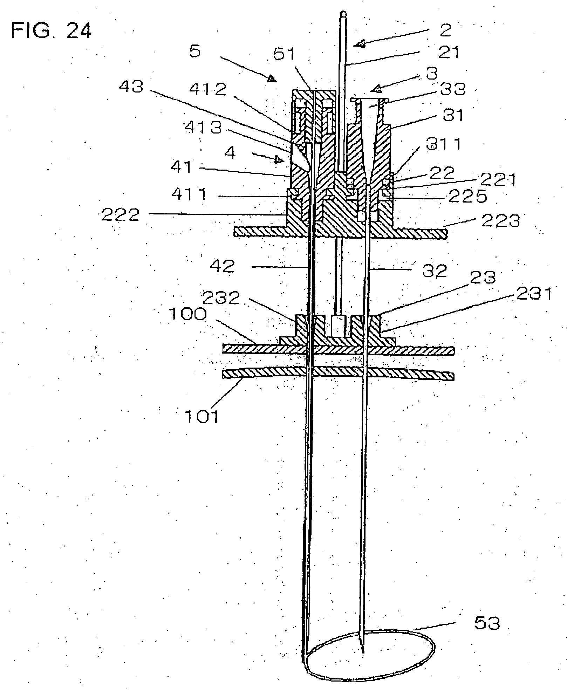

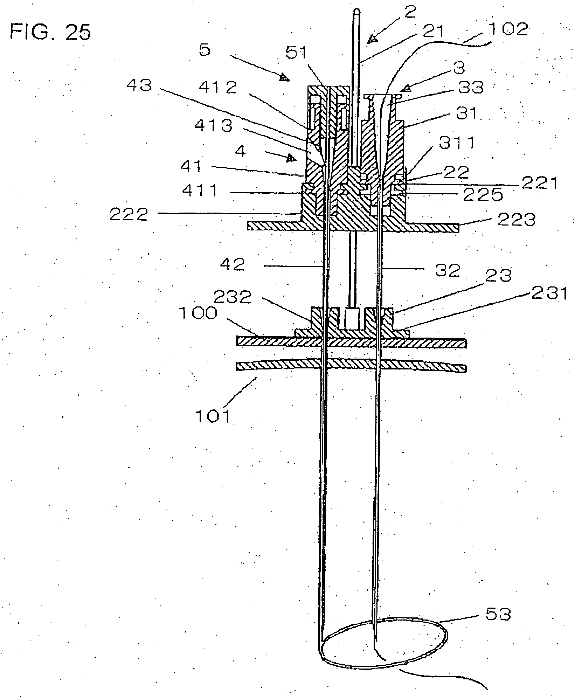

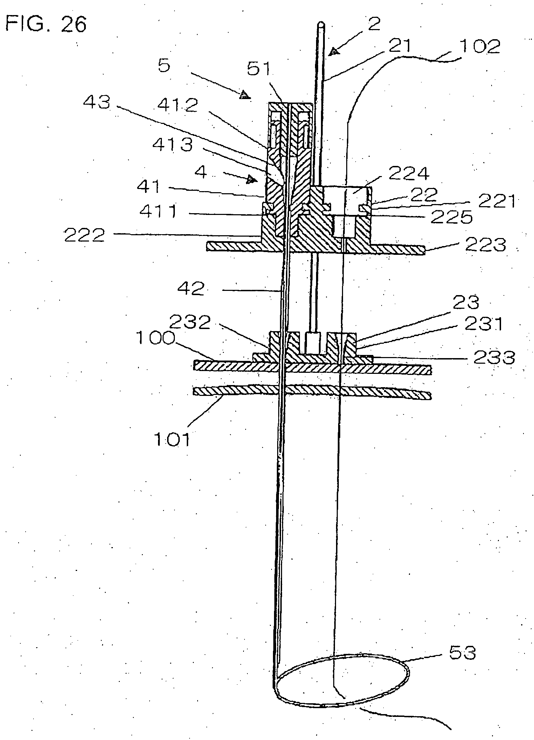

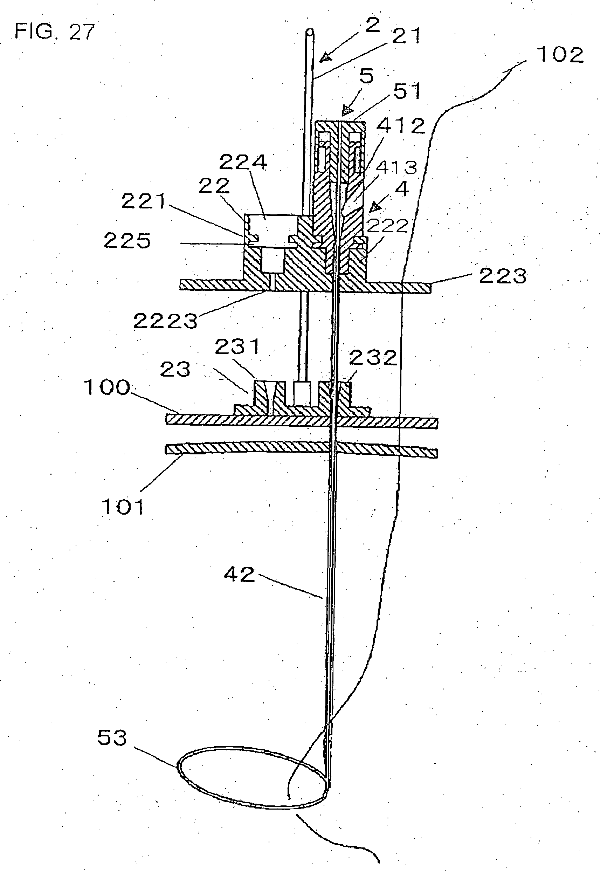

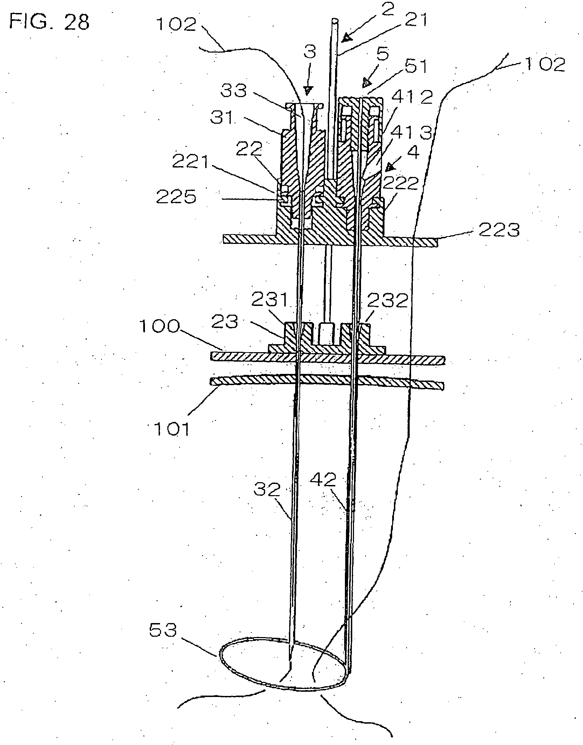

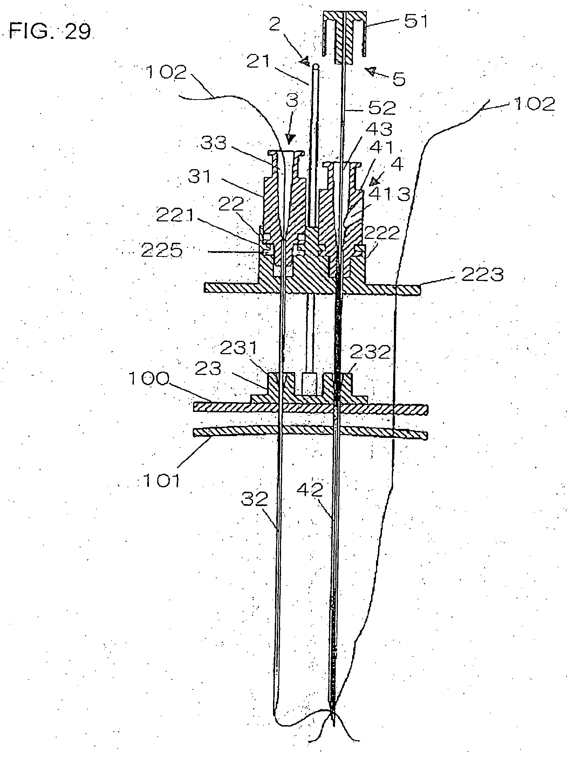

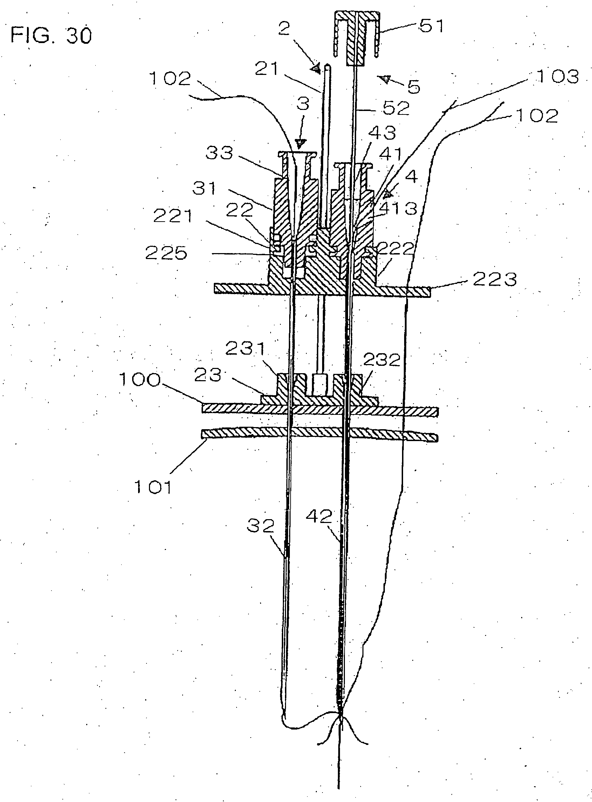

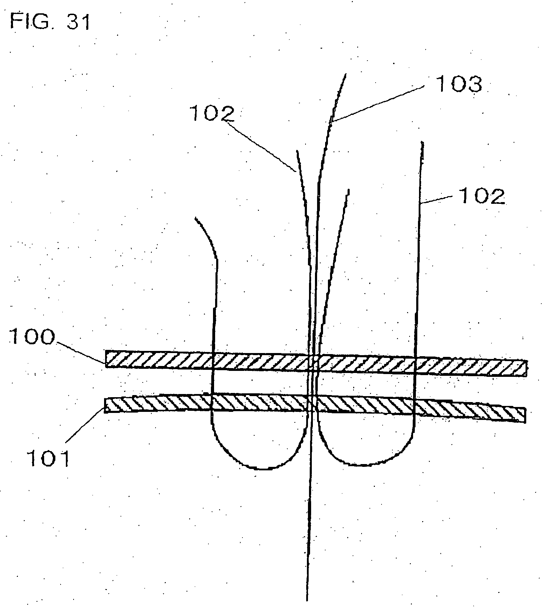

One exemplary method for using the medical device 1 will be described with reference to Figs. 22 to 31. Figs. 22 to 31 are sectional front views for describing the method for using the medical device 1. The medical device 1 can be used according to the following exemplary procedures [1] to [11].
[1] Before use of the medical device 1, the endoscope is inserted into the stomach of the patient to sufficiently supply the air thereto, and the abdominal wall 100 is closely attached to the stomach wall 101. Next, a position of the stomach is confirmed by a transparent light from the endoscope, and the abdominal skin is disinfected and anesthetized locally. A small incision is made in the position of the stomach by the knife.
[2] The abutment member 23 of the main body 2 abuts on the abdominal wall 100, and the suture gasping needle 4 punctures the abdominal wall 100 and the stomach wall 101 of the patient substantially perpendicularly from the small-incision site (Fig. 22).
[3] The handle portion 51 of the suture traction tool 5 is operated and the suture traction tool 5 is inserted into the hollow portion 43 of the suture grasping needle 4 until a lower end of the.handle portion 51 abuts on the second hub 41. The annular portion 53 of the suture traction tool 5 thereby protrudes from the tip end of the suture grasping needle 4 and spreads annually (Fig. 23). To insert the suture traction tool 5 into the suture grasping needle 4, the suture traction tool 5 may be stored in the suture grasping needle 4 in advance before performing the operation described in paragraph [2].
[4] The suture insertion needle 3 punctures the abdominal wall and the stomach wall of the patient through the fixing member 22 and the abutment member 23 substantially perpendicularly. At this time, the suture insertion needle 3 is set to pass through the inside of the annular portion 53 of the suture traction tool 5 (Fig. 24).
   In the using the exemplary method, it is unnecessary for the suture insertion needle 3 and the suture grasping needle 4 to simultaneously puncture. Since the respective puncture needle can puncture them separately, the puncture operation is easier than that when the two puncture needles are used to puncture the targets simultaneously.
[5] The suture 102 is inserted into the hollow portion 33 of the suture insertion needle 3 to protrude the suture 102 from the tip end of the suture insertion needle 3 (Fig. 25). At this time, the suture insertion needle 3 is passed through the inside of the annular portion 53, so that the suture 102 also is passed through the inside of the annular portion 53.
[6] While the main body 2 and the suture grasping needle 4 are kept as they are, only the suture insertion needle 3 is pulled out from the fixing member 22 to the base end side (upper side in Fig. 26) (Fig. 26). At this time, a protruding portion of the suture 102 can be set long. By doing so, it is possible to prevent the suture 102 from being pulled out. In addition, the suture insertion needle 3 can be pulled out while supplying the suture 102 thereto. As a result, the suture 102 is passed through the inside of the annular portion 53. At this moment, by withdrawing the suture traction tool 5, it is possible to confirm that the suture 102 is grasped.
[7] The main body 2 is rotated around the suture grasping puncture tool 4, as a rotational axis, substantially by 180 degrees (Fig. 27). By doing so, the suture 102 is passed through the-first notch 2223 of the fixing member 22 and is detached from the fixing member 22 and the abutment member 23. Care should be taken not to pull out the suture 102 from the inside of the annular portion 53.
[8] After rotation, the suture insertion needle 3 punctures the abdominal wall and the stomach wall of the patient again through the fixing member 22 and the abutment member 23 substantially perpendicularly. At this time, similarly to the operation shown in Fig. 25, the suture insertion needle 3 is set to pass through the inside of the annular portion 53 of the suture traction tool 5. The suture 102 is inserted into the hollow portion 33 of the suture insertion needle 3 to protrude the suture 102 from the tip end of the suture insertion needle 3 (Fig. 28). As a result, the two sutures 102 are arranged inside the annular portion 53.
[9] The suture traction tool 5 is slightly raised upward. At this moment, since the two sutures 102 are arranged inside the annular portion 53, the annular portion 53 enters the inner cavity of the suture grasping needle 4 and the two sutures 102 are grasped by withdrawing the suture traction tool 5 (Fig. 29).
[10] The guide wire 103 is inserted into the suture grasping needle 4 from the side port 413 of the hub 41 thereof (Fig. 30). The guide wire insertion operation may be performed at arbitrary time as long as the operation is performed after the suture grasping needle 4 punctures the abdominal wall and the stomach wall.
[11] The two sutures 102 are grasped by the suture traction tool 5, and the main body 2 is pulled out from the abdominal wall 100 while the suture insertion needle 3 is attached to the main body 2. Through these operations, the sutures 102 inserted from the two puncture positions at which the suture insertion needle 3 is inserted are passed from the abdominal wall 100 into the stomach wall 101 and protrudes from the puncture position of the suture grasping needle 4 (Fig. 31). Further, at the puncture position of the suture grasping needle 4, the guide wire 103 is inserted. Ends of the respective sutures 102 are knotted to fix the abdominal wall 100 to the stomach wall 101.

As can be understood, the medical device 1 is configured so that the suture insertion needle 3 and the suture grasping needle 4 are detachable from the main body 2. By using these puncture needles 3 and 4, it is possible to ensure putting the abdominal wall 100 to the stomach wall 101 in the two sutures 102 by the simple operation.

In this embodiment, the instance in which the suture insertion needle 3 and the suture grasping needle 4 puncture separately has been described. However, the puncture needles can simultaneously puncture. To do so, the first flange 311 of the suture insertion needle 3 may be engaged with the first flange engagement portion 225 of the first fixing portion 221, and the second flange 411 of the suture grasping needle 4 is engaged with the second flange engagement portion 227 of the second fixing portion 222.

### (THIRD EMBODIMENT)

The medical device according to the first or second embodiment can include a plurality of suture insertion needles. An instance in which the medical device according to the second embodiment includes two puncture needles will be described.

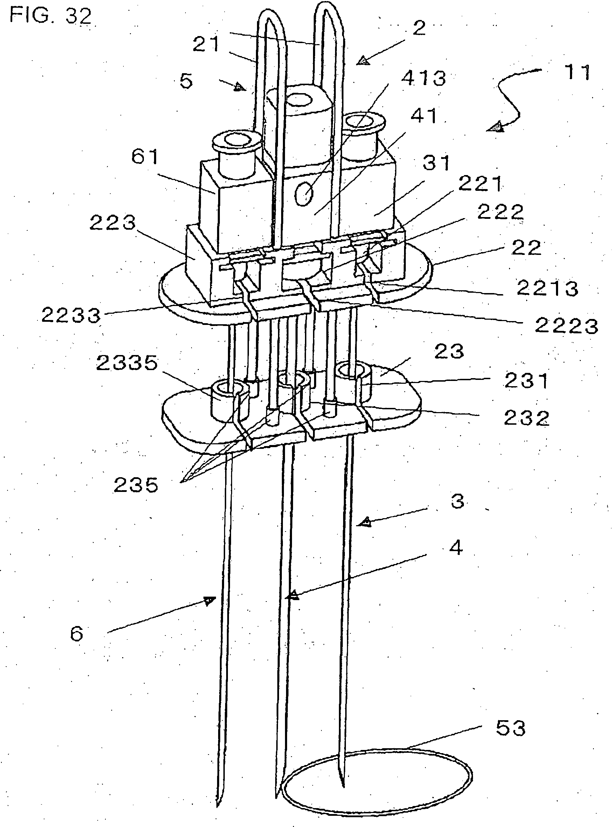

Fig. 32 is a perspective view that depicts a configuration of a medical device 11 according to the present embodiment. As shown in Fig. 32, the medical device 11 includes a main body 2, a suture insertion needle 3 and a suture insertion needle (third puncture needle) 6, which are supported by the main body 2 and into which a suture can be inserted, and a suture grasping needle 4 which is provided to be proximate to the suture insertion needles 3 and 6 and into which a suture traction tool can be inserted. The suture insertion needles 3 and 6 and the suture grasping needle 4 are detachably attached to the main body 2 by attachment and detachment means.

This medical device 11 is configured so that the suture insertion needle 6 is also detachably attached to the main body 2 of the medical device 1 (Fig. 17) according to the second embodiment. The other constituent elements can be applied to those of, for example, the medical device 1.

The respective constituent elements of the medical device 11 will be described, while centering around differences from the medical device 1 according to the second embodiment.

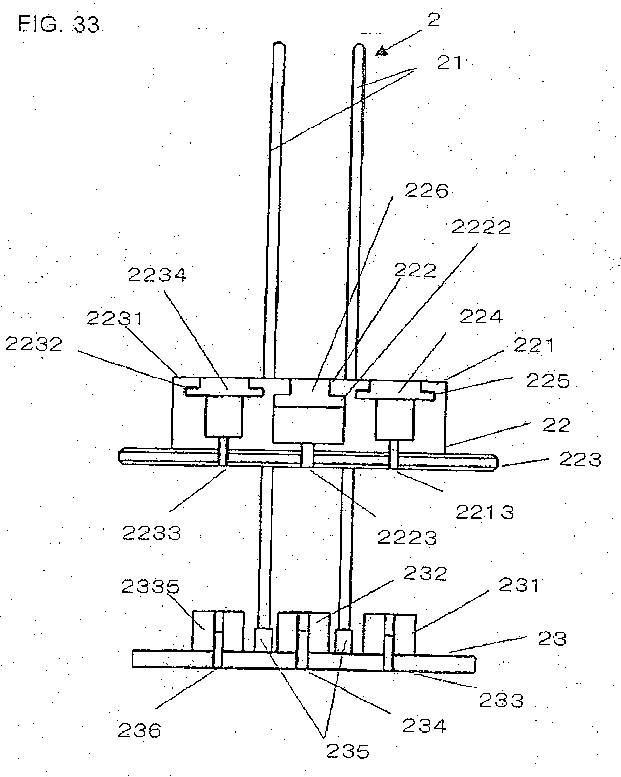

Fig. 33 is a front view that depicts a configuration of the main body 2 of the medical device 11. The main body 2 has a function of supporting the suture insertion needle 3, the suture grasping needle 4, and the suture insertion needle 6.

The main body 2 includes a support member 21, a fixing member 22 provided on a base end of the support member 21 (an upper side thereof in Fig. 33), and an abutment member 23 provided on a tip end thereof (a lower side thereof, in Fig. 33).

The fixing member 22 has a function of fixing the suture insertion needle 3, the suture grasping needle 4, and the suture insertion needle 6.

As shown in Fig. 33, the fixing member 22 includes a first fixing portion 221, a second fixing portion 222, and a third fixing portion 2231 that fix the suture insertion needle 3, the suture grasping needle 4, and the suture insertion needle 6, respectively, and a plate body 223. The first fixing portion 221, the second fixing portion 222, the third fixing portion 2231, and the plate body 223 are formed integrally.

The first fixing portion 221, the second fixing portion 222, and the third fixing portion 2231 are provided in a substantially linear manner on a base end-side surf ace (upper side in Fig. 33) of the plate body 223.

The first fixing portion 221, the second fixing portion 222, and the third fixing portion 2231 are substantially equal in structure and generally rectangular parallelepiped. The first fixing portion 221 and the third fixing portion 2231 are higher than the second fixing portion 222. It is thereby possible for a user to easily visually distinguish the respective fixing portions. In this embodiment, the first fixing portion 221 and the third fixing portion 2231 differ in height from the second fixing portion 222. Alternatively, the first fixing portion 221, the third fixing portion 2231, and the second fixing portion 222 may be equal in height.

The third fixing portion 2231 includes a third concave portion 2234 into which the suture insertion needle 6 can be inserted.

In the third concave portion 2234, a third flange engagement portion 2232 engageable with a third flange of a third hub 61 (not shown in Figs. 32 and 33), to be described later, provided on the suture insertion needle 6 is formed.

The third concave portion 2234 is hollowed out cylindrically, smaller in diameter on a tip end and larger on a base end across the third flange engagement portion 2232. The third concave portion 2234 is opened to a front surface side of the-third fixing portion 2231, so that the third hub 61 of the suture insertion needle 6, to be described later, can be inserted from the front surface side thereof.

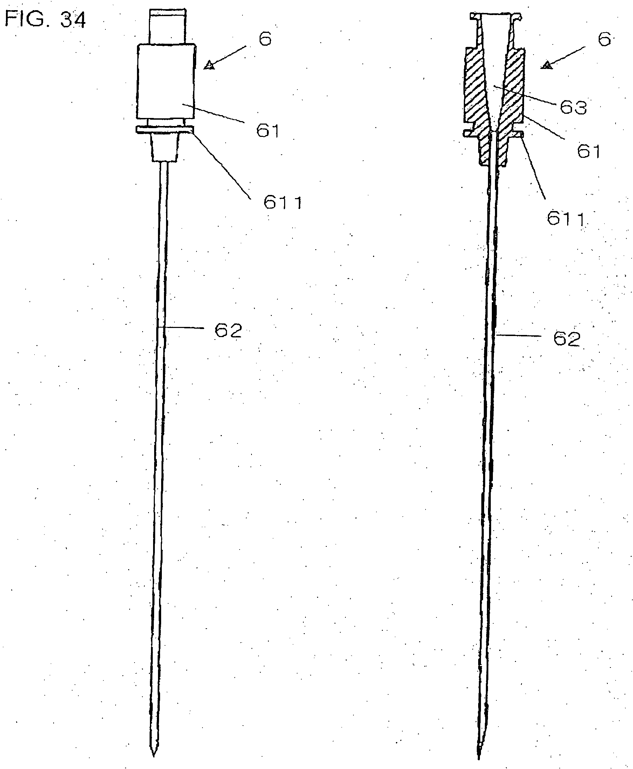

The third flange 611, to be described later with reference to Fig. 34, can be engaged with the third flange engagement portion 2232. While the third flange 611 is engaged with the third flange engagement portion 2232, the suture insertion needle 6 cannot either rotate or move vertically.

Further, the suture insertion needle 6 can be inserted into the third concave portion 2234 from the base end side without engaging the third flange 611 with the third flange engagement portion 2232. In this case, similarly to the above, an outer circumference of the third flange 611 is fitted into the third concave portion 2234 and restricted thereby, so that the suture insertion needle 6 cannot either rotate or move vertically.

A third notch 2233 is formed in the third fixing portion 2231 so that the suture insertion needle 6 is detachable. It is thereby possible to make the suture insertion needle 6 detachable, so that operativity can be improved. The third notch 2233 is formed from a front-side bottom of the fixing member 22 deep into the third concave portion 2234 through the third fixing portion 2231.

As shown in Figs. 32 and 33, the abutment member 23 is flat, and includes a first cylindrical portion 231 that includes an inner cavity into which the suture insertion needle 3 can be inserted, a second cylindrical portion 232 that includes an inner cavity into which the suture grasping needle 4 can be inserted, and a third cylindrical portion 2335 that includes an inner cavity into which the suture insertion needle 6 can be inserted, on a base end-side surface thereof. The abutment member 23 functions to improve stability when the medical device 11 is used by causing the abutment member 23 to abut on the body surface part.

The first cylindrical portion 231, the second cylindrical portion 232, and the third cylindrical portion 2335 function to improve stability when the suture insertion needle.3, the suture grasping needle 4, and the suture insertion needle 6 puncture, respectively.

The abutment member 23 includes a first notch 233, a second notch 234, and a third notch 236 serving as the attachment and detachment means so that the suture insertion needle 3, the suture grasping needle 4, and the suture insertion needle 6 are detachable to the abutment member 23, respectively. The suture insertion needle 3, the suture grasping needle 4, and the suture insertion needle 6 can thereby be detachably hold, thus improving the operativity.

The first notch 233, the second notch 234, and the third notch 236 are formed from bottom portions of the abutment member 23 deep into the first cylindrical portion 231, the second cylindrical portion 232, and the third cylindrical portion 2335 therethrough, respectively. The suture insertion needle 3, the suture grasping needle 4, and suture insertion needle 6 are thereby made detachable to the abutment member 23.

Fig. 34 depicts a front view and a sectional view of the suture insertion needle 6. As shown in Fig. 34, the suture insertion needle 6 includes the third hub 61 provided on.a base end thereof (upper side in Fig. 34) and a third needle portion 62 provided on a tip end thereof. The suture insertion needle 6 functions to insert the suture into an internal tissue, similarly to the suture insertion needle 3.

The third hub 61 and the third needle portion 62 include a hollow portion 63, which serves as a passage of the suture. The hollow portion 63 within the third hub 61 is formed so that the inside diameter is gradually smaller from a base end to a tip end.

In this embodiment, the third hub 61 corresponds to the stopper 719 (Fig. 3) of the medical device according to the first embodiment.

The third hub 61 is generally cylindrical and includes a third flange 611 engageable with the third flange engagement portion 2232 on a tip end thereof.

The third needle portion 62 includes an opening on a tip end thereof (lower side in Fig. 34) to communicate with the hollow portion 63. The third needle portion 62 keeps a constant outside diameter from a base end near to a tip end and has a sharp needle point formed on the tip end.

The configuration of the first needle portion 32 can be applied to that of the third needle portion 62. A shape of the third needle portion 62 can be designed equally to, for example, that of the suture insertion needle 715 (Fig. 4) according to the first embodiment. By so designing, in the third needle portion 62, it is possible to ensure that a lateral hole communicating with the outside is oriented to the second needle portion 42. In addition, the suture can be smoothly inserted into the hollow portion 63.

An outside diameter of the third needle portion 62 is not limited to a specific diameter; however, it can be set, for example, equal to or larger than 15G and equal to or smaller than 25G, preferably equal to or larger than 19G and equal to or smaller than 21G. By so setting, it is possible to reduce a puncture resistance of the suture insertion needle 6 and the suture insertion needle 6 can easily puncture the body.

An entire length of the third needle portion 62 is not limited to a specific length; however, it can be set, for example, equal to or larger than 20 mm and equal to or smaller than 200 mm, preferably equal to or larger than 80 mm and equal to or smaller than 100 mm. If the entire length is too small, it may possibly be buried in the abdominal wall of the patient. If too large, the operativity of the medical device 11 may possibly be deteriorated.

Another exemplary method for using the medical device 11 will be described. As stated above, the respective constituent elements of the medical device 1 are basically applied to the medical device 11, and the medical device 11 is configured so that the suture insertion needle 6 can be further detachably provided on the main body 2 of the medical device 1. When using the medical device 11, therefore, the exemplary procedures for using the medical device 1 can be adopted. Differences from the exemplary procedures described in the paragraphs [1] to [11] will be mainly described hereafter.
[12] The exemplary operations described from [1] to [5] by the medical device 1 are similarly carried out.
[13] While the main body 2, the suture insertion needle 3, and the suture grasping needle 4 are kept as they are, the handle portion 51 of the suture traction tool 5 is operated to rotate the suture traction tool 5 by 180 degrees. Care should be taken not to pull out the suture 102 from the inside of the annular portion 53. At this moment, a protrusion of the suture 102 can be made long so as not to pull out the suture 102.
[14] The suture insertion needle 6 punctures the abdominal wall and the stomach wall of the patient through the fixing member 22 and the abutment member 23 substantially perpendicularly. At this time, similarly to the operation shown in Fig. 25, the suture insertion needle 6 is set to pass through the inside of the annular portion 53 of the suture traction tool 5. Then the suture 102 is inserted into the hollow portion 63 of the suture insertion needle 6 to protrude the suture 102 from the tip end of the suture insertion needle 6. As a result, the two sutures 102 are arranged inside the annular portion 53.
   In this exemplary method, it is unnecessary for the suture insertion needle 3, the suture grasping needle 4, and the suture insertion needle 6 to puncture simultaneously. Namely, the respective puncture needles can puncture them separately, so that puncture operation can be performed easily as compared with the instance in which the two puncture needles puncture simultaneously.
[15] The suture traction tool 5 is slightly raised upward. At this moment, since the two sutures 102 are arranged inside the annular portion 53, the annular portion 53 enters the inner cavity of the suture grasping needle 4 and the two sutures 102 are grasped by withdrawing the suture traction tool 5.
[16] The guide wire 103 is inserted into the suture grasping needle 4 from the side port 413 of the hub 41 thereof. The guide wire insertion operation may be performed at arbitrary time as long as the operation is performed after the suture grasping needle 4 punctures.
[17] The main body 2 is pulled out from the abdominal wall 100 while the two sutures 102 are grasped by the suture traction tool 5 and the suture insertion needles 3 and 6 are attached to the main body 2. Through these operations, the sutures 102 inserted from the two puncture positions at which the suture insertion needles 3 and 6 are inserted are passed from the abdominal wall 100 into the stomach wall 101 and protrudes from the puncture position of the suture grasping needle 4. Further, at the puncture position of the suture grasping needle 4, the guide wire 103 is inserted. Ends of the respective sutures 102 are knotted to fix the abdominal wall 100 to the stomach wall 101.

As can be understood, by using the medical device 1.1, it is possible to further ensure putting the abdominal wall 100 to the stomach wall 101 in the two sutures 102.

For example, the shapes of the support member, the fixing member, the abutment member, and so forth of the main body, the structure of the attachment and detachment means, the shapes of the hubs of the respective puncture needles, or the like may differ from those according to the disclosed embodiments. Specifically, a hole into which the guide wire 103 can be inserted may be provided on the upper surface of the handle portion 51. If this hole communicates with the hollow portion 43 in the second hub 41, the suture traction tool 5 and the guide wire 103 can be easily inserted into the suture grasping needle 4 simultaneously. The hole into which the guide wire 103 can be inserted and which is formed in the handle portion 51 may be formed in an arbitrary portion of the handle portion 51.

Further, the suture grasping needle 4 or 701 may include two or more lumens. If so, the simultaneous insertion of the suture traction tool 5 and the guide wire 103 into the suture grasping needle 4 can be easily performed.

Moreover, the target sites of the living body are not limited to the abdominal wall and the stomach wall. The present invention is also applicable to lifting or the like of various visceral walls, blood vessels, nerves, and the like. to the abdominal wall.

## Claims

1. A medical device used for suturing a body surface part and an internal tissue, comprising:
(a) a suture insertion needle (715) that is a hollow puncture needle including a sharp tip end (716), said tip end being non-hollow, that includes a lateral hole (718) near said tip end, and that is used by inserting a suture (705) into a hollow portion (717) of said suture insertion needle;
(b) a suture grasping needle (701) that is a hollow puncture needle arranged sideways of said suture insertion needle (715) substantially in parallel to said suture insertion needle and including a sharp tip end (702), said tip end being non-hollow, that includes a lateral hole (704) near the tip end, and that is used by inserting said suture (705) into a hollow portion (703) of said suture grasping needle;
(c) a suture traction tool (710) slidably inserted into said hollow portion (703) of said suture grasping needle (701);
(d) a fixing member (708) for relatively determining a tip end position of said suture insertion needle (715) and a tip end position of said suture grasping needle (701);
(e) an abutment member (722) that supports said suture insertion needle (715) and said suture grasping needle (701), and that abuts on said body surface part; and
(f) a notch (723) being formed from a side edge deep into said abutment member (722),
wherein at least one of said suture insertion needle (715) and said suture grasping needle (701) supported by said abutment member (722) is laterally detachable from said abutment member through said notch.

2. The medical device according to claim 1,
wherein said suture traction tool (710) includes a snare (712) provided on a tip end of said suture traction tool (710) and formed out of an elastically deformable material, which extends so that an extension of said suture insertion needle (715) passes through an inside of said snare (712) in a free state, and can be stored in said hollow portion (703) of said suture grasping needle (701) in an elastically deformed state.

3. The medical device according to claim 1,
wherein said abutment member (722) supports said suture insertion needle (715) and said suture grasping needle (701) substantially parallel to each other.

4. The medical device according to claim 1,
wherein said fixing member (708) is provided at said suture grasping needle (701), and configured to be able to determine the tip end position of said suture insertion needle (715) relative to the tip end position of said suture grasping needle (701), to an arbitrary position.

5. The medical device according to claim 1,
wherein a notch configured to remove said suture (705) from said fixing member (708) is formed from a side edge deep into said fixing member (708).

6. The medical device according to claim 1, wherein
an edge of said lateral hole (718) of said suture insertion needle (715) and an edge of said lateral hole (704) of said suture grasping needle (701) are chamfered to have a C-shaped surface or an R-shaped surface.

7. The medical device according to claim 1,
wherein at least one of said suture insertion needle (715) and said suture grasping needle (701) is detachably supported by said abutment member (722).

8. The medical device according to claim 1, further comprising a plurality of said suture insertion needles,
wherein said suture grasping needle (701) is provided to be proximate to each of said plurality of said suture insertion needles.

9. A medical device used for suturing a body surface part and an internal tissue, comprising:
a main body (2);
a suture insertion needle (3, 6) which is supported by said main body (2), and into which the suture can be inserted;
a suture grasping needle (4) which is provided to be proximate to said suture insertion needle (3, 6), and into which a suture traction tool (5) can be inserted;
an abutment member (23) that is integrally provided on a tip end side of said main body (2), that abuts on said body surface part, and that supports said suture insertion needle (3, 6) and said suture grasping needle (4); and
a notch (233, 234, 236) being formed from a side edge deep into said abutment member (23),
wherein at least one of said suture insertion needle (3, 6) and said suture grasping needle (4) supported by said abutment member (23) is laterally detachable from said abutment member (23) through said notch.

10. The medical device according to claim 9, wherein said main body includes:
a fixing member (22) that fixes one of a tip end of said suture insertion needle (3, 6) and a tip end of said suture grasping needle (4) to a predetermined position; and
said main body (2) comprises a support member (21) that integrally supports said fixing member (22) and said abutment member (23),
wherein said fixing member (22) includes a notch (2213, 2223, 2233) being formed from a side edge deep into said fixing member (22) and making one of said suture insertion needle (3, 6) and said suture grasping needle (4) laterally detachable from said fixing member (22).

11. The medical device according to claim 9,
wherein said suture grasping needle (4) is configured so that a guide wire (103) can be also inserted into said suture grasping needle (4).

12. The medical device according to claim 11,
wherein an outside diameter of said guide wire is equal to or larger than 0.1 mm and equal to or smaller than 0.8 mm.

13. The medical device according to claim 9,
wherein said suture insertion needle (3, 6) and said suture grasping needle (4) are configured to be able to puncture independently of each other.

14. The medical device according to claim 9,
wherein while said suture insertion needle (3, 6) and said suture grasping needle (4) puncture and said main body (2) abuts on said body surface part, said suture insertion needle (3, 6) can be independently removed.

15. The medical device according to claim 9,
wherein said main body (2) is rotatable about said suture grasping needle (4) used as a rotational axis:

16. The medical device according to claim 9, further comprising a plurality of said suture insertion needles,
wherein said suture grasping needle (4) is provided to be proximate to each of the plurality of suture insertion needles (3, 6).

## Patentansprüche

1. Medizinische Vorrichtung, die zum Vernähen eines Körperflächenteils und eines inneren Gewebes verwendet wird, umfassend:
(a) eine Fadeneinbringungsnadel (715), die eine hohle Punktionsnadel ist, die ein scharfes Spitzenende (716) umfasst, wobei das Spitzenende nicht-hohl ist, die ein laterales Loch (718) nahe dem Spitzenende umfasst, und die durch Einbringen eines Fadens (705) in einen hohlen Bereich (717) der Fadeneinbringungsnadel verwendet wird;
(b) eine Fadengreifnadel (701), die eine hohle Punktionsnadel ist, die seitlich von der Fadeneinbringungsnadel (715) angeordnet ist, im Wesentlichen parallel zu der Fadeneinbringungsnadel, und ein scharfes Spitzenende (702) umfasst, wobei das Spitzenende nicht-hohl ist, die ein laterales Loch (704) nahe dem Spitzenende umfasst, und die durch Einbringen des Fadens (705) in einen hohlen Bereich (703) der Fadengreifnadel verwendet wird;
(c) ein Faden-Zug-Werkzeug (710), das gleitbar in den hohlen Bereich (703) der Fadengreifnadel (701) eingebracht ist;
(d) ein Befestigungsteil (708) zum relativen Feststellen einer Spitzenenden-Position der Fadeneinbringungsnadel (715) und einer Spitzenenden-Position der Fadengreifnadel (701);
(e) ein Widerlagerteil (722), das die Fadeneinbringungsnadel (715) und die Fadengreifnadel (701) stützt, und das an den Körperflächenteil angrenzt; und
(f) einen Einschnitt (723), der von einer Seitenkante tief in das Widerlagerteil (722) hinein gebildet ist,
wobei die Fadeneinbringungsnadel (715) und/oder die Fadengreifnadel (701), die von dem Widerlagerteil (722) gestützt ist/sind, lateral von dem Widerlagerteil durch den Einschnitt herausnehmbar ist/sind.

2. Medizinische Vorrichtung nach Anspruch 1,
wobei das Faden-Zug-Werkzeug (710) eine Schlinge (712) umfasst, die an einem Spitzenende des Faden-Zug-Werkzeugs (710) bereitgestellt ist und aus einem elastisch deformierbaren Material ausgebildet ist, welches sich erweitert, so dass eine Ausdehnung der Fadeneinbringungsnadel (715) durch ein Inneres der Schlinge (712) in einem freien Status hindurch läuft, und in dem hohlen Bereich (703) der Fadengreifnadel (701) in einem elastisch deformierten Status gelagert werden kann.

3. Medizinische Vorrichtung nach Anspruch 1,
wobei das Widerlagerteil (722) die Fadeneinbringungsnadel (715) und die Fadengreifnadel (701) im Wesentlichen parallel zueinander stützt.

4. Medizinische Vorrichtung nach Anspruch 1,
wobei das Befestigungsteil (708) an der Fadengreifnadel (701) bereitgestellt ist, und ausgebildet ist, fähig zu sein, die Spitzenenden-Position der Fadeneinbringungsnadel (715) relativ zu der Spitzenenden-Position der Fadengreifnadel (701) an einer beliebigen Position festzustellen.

5. Medizinische Vorrichtung nach Anspruch 1,
wobei ein Einschnitt, der ausgebildet ist, den Faden (705) von dem Befestigungsteil (708) zu entfernen, von einer Seitenkante tief in das Befestigungsteil (708) hinein gebildet ist.

6. Medizinische Vorrichtung nach Anspruch 1,
wobei eine Kante des lateralen Lochs (718) der Fadeneinbringungsnadel (715) und eine Kante des lateralen Lochs (704) der Fadengreifnadel (701) abgeschrägt sind, um eine C-förmige Fläche oder eine R-förmige Fläche aufzuweisen.

7. Medizinische Vorrichtung nach Anspruch 1,
wobei die Fadeneinbringungsnadel (715) und/oder die Fadengreifnadel (701) herausnehmbar durch das Widerlagerteil (722) gestützt ist/sind.

8. Medizinische Vorrichtung nach Anspruch 1, ferner umfassend eine Vielzahl der Fadeneinbringungsnadeln,
wobei die Fadengreifnadel (701) bereitgestellt ist, nahegelegen zu jeder der Vielzahl der Fadeneinbringungsnadeln zu sein.

9. Medizinische Vorrichtung, die zum Vernähen eines Körperflächenteils und eines inneren Gewebes verwendet wird, umfassend:
einen Hauptkörper (2);
eine Fadeneinbringungsnadel (3, 6), die durch den Hauptkörper (2) gestützt ist, und in welche der Faden eingebracht werden kann;
eine Fadengreifnadel (4), die bereitgestellt ist, nahegelegen zu der Fadeneinbringungsnadel (3, 6) zu sein, und in welche ein Faden-Zug-Werkzeug (5) eingebracht werden kann;
ein Widerlagerteil (23), das integral an einer Spitzenendenseite des Hauptkörpers (2) bereitgestellt ist, das an den Körperflächenteil angrenzt, und das die Fadeneinbringungsnadel (3, 6) und die Fadengreifnadel (4) stützt; und
ein Einschnitt (233, 234, 236), der von einer Seitenkante tief in das Widerlagerteil (23) hinein gebildet ist,
wobei die Fadeneinbringungsnadel (3, 6) und/oder die Fadengreifnadel (4), die von dem Widerlagerteil (23) gestützt ist/sind, lateral von dem Widerlagerteil (23) durch den Einschnitt herausnehmbar ist/sind.

10. Medizinische Vorrichtung nach Anspruch 9, wobei der Hauptkörper umfasst:
ein Befestigungsteil (22), das ein Spitzenende der Fadeneinbringungsnadel (3, 6) oder ein Spitzenende der Fadengreifnadel (4) an einer vorbestimmten Position befestigt; und
wobei der Hauptkörper (2) ein Stützteil (21) umfasst, das das Befestigungsteil (22) und das Widerlagerteil (23) integral stützt,
wobei das Befestigungsteil (22) einen Einschnitt (2213, 2223, 2233) umfasst, der von einer Seitenkante tief in das Befestigungsteil (22) hinein gebildet ist, und die Fadeneinbringungsnadel (3, 6) oder die Fadengreifnadel (4) lateral von dem Befestigungsteil (22) herausnehmbar macht.

11. Medizinische Vorrichtung nach Anspruch 9,
wobei die Fadengreifnadel (4) so ausgebildet ist, dass auch ein Führungsdraht (103) in die Fadengreifnadel (4) eingebracht werden kann.

12. Medizinische Vorrichtung nach Anspruch 11,
wobei ein Außendurchmesser des Führungsdrahts gleich oder größer als 0,1 mm und gleich oder kleiner als 0,8 mm ist.

13. Medizinische Vorrichtung nach Anspruch 9,
wobei die Fadeneinbringungsnadel (3, 6) und die Fadengreifnadel (4) ausgebildet sind, fähig zu sein, unabhängig voneinander zu punktieren.

14. Medizinische Vorrichtung nach Anspruch 9,
wobei, während die Fadeneinbringungsnadel (3, 6) und die Fadengreifnadel (4) punktieren und der Hauptkörper (2) an den Körperflächenteil angrenzt, die Fadeneinbringungsnadel (3, 6) unabhängig entfernt werden kann.

15. Medizinische Vorrichtung nach Anspruch 9,
wobei der Hauptkörper (2) rotierbar um die Fadengreifnadel (4), die als eine Rotationsachse verwendet wird, ist.

16. Medizinische Vorrichtung nach Anspruch 9, die ferner eine Vielzahl der Fadeneinbringungsnadeln umfasst,
wobei die Fadengreifnadel (4) bereitgestellt ist, nahegelegen zu jeder der Vielzahl der Fadeneinbringungsnadeln (3, 6) zu sein.

## Revendications

1. Instrument médical utilisé pour la suture d'une partie de surface corporelle et d'un tissu interne, comprenant:
(a) une aiguille d'insertion de suture (715) qui est une aiguille de perçage creuse comprenant une extrémité à pointe tranchante (716), ladite extrémité en pointe n'étant pas creuse, comportant un trou latéral (718) proche de ladite extrémité en pointe, et qui est utilisée pour l'insertion d'un fil de suture (705) dans une portion creuse (717) de ladite aiguille d'insertion de suture;
(b) une aiguille de préhension de suture (701) qui est une aiguille de perçage creuse agencée latéralement par rapport à ladite aiguille d'insertion d'une suture (715) substantiellement parallèlement à ladite aiguille d'insertion de suture et comportant une extrémité à pointe tranchante (702), ladite extrémité en pointe étant non-creuse, comportant un trou latéral (704) proche de l'extrémité en pointe, et qui est utilisée pour l'insertion dudit fil de suture (705) dans une portion creuse (703) de ladite aiguille de préhension de suture;
(c) un outil de traction de suture (710) inséré avec faculté de coulissement dans ladite portion creuse (703) de ladite aiguille de préhension de suture (701);
(d) un élément de fixation (708) pour la détermination relative d'une position de l'extrémité en pointe de ladite aiguille d'insertion d'une suture (715) et d'une position de l'extrémité en pointe de ladite aiguille de préhension de suture (701); et
(e) un élément de butée (722) qui soutient ladite aiguille d'insertion d'une suture (715) et ladite aiguille de préhension de suture (701), et qui bute sur ladite partie de surface du corps; et
(f) une rainure (723) qui est formée à partir d'un bord latéral dans la profondeur dudit élément de butée (722),
tandis que au moins une parmi ladite aiguille d'insertion d'une suture (715) et ladite aiguille de préhension de suture (701) supportée par ledit élément de butée (722) est apte à être détachée latéralement dudit élément de butée par l'intermédiaire de ladite rainure.

2. Instrument médical selon la revendication 1,
dans lequel ledit outil de traction de suture (710) comporte un collet (712) disposé sur une extrémité en pointe dudit outil de traction de suture (710) et formé à partir d'une matière déformable élastiquement, qui s'étend de telle manière qu'une extension de ladite aiguille d'insertion d'une suture (715) passe à travers un espace intérieur dudit collet (712) dans un état libre, et peut être conservée dans ladite portion creuse (703) de ladite aiguille de préhension de suture (701) dans un état déformé élastiquement.

3. Instrument médical selon la revendication 1,
dans lequel ledit élément de butée (722) supporte ladite aiguille d'insertion de suture (715) et ladite aiguille de préhension de suture (701) substantiellement parallèlement l'une par rapport à l'autre.

4. Instrument médical selon la revendication 1,
dans lequel ledit élément de fixation (708) est procuré à ladite aiguille de préhension de suture (701), et configuré pour permettre de déterminer la position de l'extrémité à pointe de ladite aiguille d'insertion de suture (715) par rapport à la position de l'extrémité à pointe de ladite aiguille de préhension de suture (701), pour une position arbitraire.

5. Instrument médical selon la revendication 1,
dans lequel une rainure configurée pour permettre d'enlever ledit fil de suture (705) dudit élément de fixation (708) est formée à partir d'un bord latéral dans la profondeur dudit élément de fixation (708).

6. Instrument médical selon la revendication 1,
dans lequel un bord dudit trou latéral (718) de ladite aiguille d'insertion de suture (715) et un bord dudit trou latéral (704) de ladite aiguille de préhension de suture (701) sont chanfreinés pour avoir une surface en forme de C ou une surface en forme de R.

7. Instrument médical selon la revendication 1,
dans lequel au moins l'une parmi ladite aiguille d'insertion de suture (715) et ladite aiguille de préhension de suture (701) est supportée de manière détachable par ledit élément de butée (722).

8. Instrument médical selon la revendication 1, comportant en outre une pluralité desdites aiguilles d'insertion de suture,
tandis que ladite aiguille de préhension de suture (701) est agencée pour être à proximité de chacune parmi ladite pluralité desdites aiguilles d'insertion de suture.

9. Instrument médical utilisé pour suturer une partie de surface corporelle et un tissu interne, comprenant:
un corps principal (2);
une aiguille d'insertion de suture (3, 6) qui est supportée par ledit corps principal (2), et dans laquelle la suture peut être insérée;
une aiguille de préhension de suture (4) qui est agencée pour être à proximité de ladite aiguille d'insertion de suture (3, 6) et dans laquelle peut être inséré un outil de traction de suture (5);
un élément de butée (23) qui fait partie intégrante d'un côté d'extrémité en pointe dudit corps principal (2), qui bute contre ladite partie de surface corporelle, et qui supporte ladite aiguille d'insertion de suture (3, 6) et ladite aiguille de préhension de suture (4); et
une rainure (233, 234, 236) étant formée à partir d'un bord latéral dans la profondeur dudit élément de butée (23),
tandis que au moins l'une parmi ladite aiguille d'insertion de suture (3, 6) et ladite aiguille de préhension de suture (4) supportée par ledit élément de butée (23) est détachable latéralement dudit élément de butée (23) par l'intermédiaire de ladite rainure.

10. Instrument médical selon la revendication 9, dans lequel ledit corps principal comprend:
un élément de fixation (22) qui fixe l'une parmi une extrémité en pointe de ladite aiguille d'insertion de suture (3, 6) et une extrémité en pointe de ladite aiguille de préhension de suture (4) dans une position prédéterminée; et
ledit corps principal (2) comprend un élément support (21) qui supporte intégralement ledit élément de fixation (22) et ledit élément de butée (23),
tandis que ledit élément de fixation (22) comprend une rainure (2213, 2223, 2233) qui est formée à partir d'un bord latéral dans la profondeur dudit élément de fixation (22), et que l'une parmi ladite aiguille d'insertion de suture (3, 6) et ladite aiguille de préhension de suture (4) est rendue détachable latéralement à partir dudit élément de fixation (22).

11. Instrument médical selon la revendication 9,
dans lequel ladite aiguille de préhension de suture (4) est configurée de telle manière qu'un fil guide (103) peut être également inséré dans ladite aiguille de préhension de suture (4).

12. Instrument médical selon la revendication 11,
dans lequel un diamètre externe dudit fil guide est égal ou supérieur à 0,1 mm et égal ou inférieur à 0,8 mm.

13. Instrument médical selon la revendication 9,
dans lequel ladite aiguille d'insertion de suture (3, 6) et ladite aiguille de préhension de suture (4) sont configurées pour être aptes à percer indépendamment l'une de l'autre.

14. Instrument médical selon la revendication 9,
dans lequel, tandis que ladite aiguille d'insertion de suture (3, 6) et ladite aiguille de préhension de suture (4) percent et que ledit corps principal (2) est en butée sur ladite partie de surface corporelle, ladite aiguille d'insertion de suture (3, 6) peut être retirée indépendamment.

15. Instrument médical selon la revendication 9,
dans lequel ledit corps principal (2) peut être mis en rotation autour de ladite aiguille de préhension de suture (4) utilisée comme axe de rotation.

16. Instrument médical selon la revendication 9, comprenant en outre une pluralité desdites aiguilles d'insertion de suture,
tandis que ladite aiguille de préhension de suture (4) est agencée pour être à proximité de chacune parmi la pluralité d'aiguilles d'insertion de suture (3, 6).
